Europäisches Patentamt

(19) European Patent Office

Office européen des brevets

(11) Numéro de publication : **0 236 210 B1**

(12)
# FASCICULE DE BREVET EUROPEEN

(45) Date de publication du fascicule du brevet :
**23.10.91 Bulletin 91/43**

(51) Int. Cl.$^5$: **C12N 15/14**, C12N 15/62,
C12N 15/70

(21) Numéro de dépôt : **87400355.1**

(22) Date de dépôt : **19.02.87**

(54) **Procédé de préparation de la sérum albumine humaine mature.**

(30) Priorité : **21.02.86 FR 8602379**

(43) Date de publication de la demande :
**09.09.87 Bulletin 87/37**

(45) Mention de la délivrance du brevet :
**23.10.91 Bulletin 91/43**

(84) Etats contractants désignés :
**AT BE CH DE FR GB IT LI LU NL SE**

(56) Documents cités :
EP-A- 0 001 929
EP-A- 0 073 646
EP-A- 0 114 506
EP-A- 0 138 437
EP-A- 0 200 590
THE EMBO JOURNAL, vol. 3, no. 6, juin 1984,
pages 1429-1434, IRL Press Ltd, Oxford, GB;
K.K. STANLEY et al.: "Construction of a new
family of high efficiency bacterial expression
vectors: identification of cDNA clones coding
for human liver proteins"

(73) Titulaire : **GENETICA**
**160 Quai de Polangis**
**94340 Joinville Le Pont (FR)**

(72) Inventeur : **Latta, Martine**
**297 Rue de Charenton-75**
**F-75012 Paris (FR)**
Inventeur : **Mayaux, Jean-François**
**2Iter, Boulevard de la République**
**F-92260 Fontenay aux Roses (FR)**
Inventeur : **Sarmientos, Paolo**
**Via Mose Bianchi 104**
**Milano (IT)**

(74) Mandataire : **Pilard, Jacques et al**
**RHONE-POULENC INTERSERVICES Service**
**Brevets Pharma 25, Quai Paul Doumer**
**F-92408 Courbevoie Cédex (FR)**

## Description

La présente invention concerne un procédé de préparation de la sérum-albumine humaine mature à partir d'une sérum-albumine humaine produite par voie microbiologique sous forme de protéine fusionnée.

Il existe un grand choix d'organismes hôtes, tels que les cellules mammifères modifiées ou les micro-organismes qui peuvent potentiellement être utilisés en vue de la production en quantités importantes de protéines humaines d'une grande valeur thérapeutique.

L'utilisation de cellules mammifères modifiées par les techniques de l'ADN recombinant présente l'avantage de conduire à des produits très proches de ceux d'origine naturelle ; cependant la culture de ces cellules est délicate et ne peut être conduite que dans des volumes limités.

L'emploi de micro-organismes, tels que les bactéries, permet une fabrication à une échelle plus importante mais présente l'inconvénient de conduire à des produits qui diffèrent sensiblement des produits d'origine naturelle. Ainsi les protéines normalement glycosylées chez l'homme ne sont pas, en général, glycosylées par les bactéries [P. Berman et L.A. Laskey, Trends Biochem. Sci., (1985) 10, p.51 et suivantes]. Par ailleurs, les protéines humaines exprimées à haut niveau dans des bactéries telles que E.coli acquièrent souvent une conformation non native qui s'accompagne d'une précipitation intracellulaire [R.G. Schoner et coll., Bio. Technol. (1985), 3, p.151 et suivantes ; J.M. Schoemaker et coll., EMBO J. (1985), 4, p.775 et suivantes]. Enfin, pour qu'un gène puisse s'exprimer dans une bactérie, telle que E.coli, il est indispensable de positionner un codon initiateur méthionine devant la séquence codante de la protéine mature. Généralement, ce résidu n'est pas excisé par la méthionyl aminopeptidase de E.coli [P.H. Seeburg et coll., 1985, 2, p.37 et suivantes ; J.M. Schoner et coll., Proc. Natl. Acad. Sci. USA (1981), 81, p.5403]. La protéine obtenue présente donc un acide aminé anormal comme premier résidu qui peut provoquer l'inhibition stérique d'une activité biologique si le début de la protéine est impliqué dans cette activité. Le résidu peut également présenter un caractère immunogène néfaste à l'administration ultérieure de la protéine.

Il résulte que le choix d'une cellule-hôte dépend de la protéine spécifique que l'on veut obtenir. Dans le cas d'une protéine de valeur marchande élevée et nécessaire en quantité limitée, les cellules mammifères peuvent constituer une source particulièrement bien adaptée. Par contre dans le cas d'un produit de valeur marchande plus faible et nécessaire en quantité importante, de l'ordre de plusieurs dizaines de tonnes, telle que la sérum-albumine humaine (SAH), il paraît indispensable d'utiliser des micro-organismes tout en remédiant aux inconvénients liés à leur emploi.

Lorsque la SAH est exprimée à partir d'une construction génétique du type "Promoteur-Site de démarrage de traduction-ATG-Gène de la SAH mature", la protéine obtenue conserve généralement une méthionine comme résidu N-terminal. Pour éliminer la méthionine N-terminale de protéines hétérologues exprimées chez E.coli, plusieurs méthodes peuvent être envisagées, telles que le clivage enzymatique in vivo, l'excision protéolytique pendant ou immédiatement après le transport à travers la membrane ou bien des digestions protéolytiques ou chimiques in vivo.

Il est connu, en particulier d'après J.P. Waller, J. Mol. Biol., (1963), 7, p.483 et suivantes, que E.coli possède une méthionyl aminopeptidase qui excise la méthionine N-terminale d'un certain nombre de protéines. Cependant la spécificité du mécanisme est mal connue et il est supposé que ce mécanisme dépend du ou des résidus suivant la méthionine [V.M. Vogt, J. Biol. Chem. (1970), 245, p.4760 et suivantes ; H.J. George et coll., (1985) DNA, 4, p.273].

Les protéines sécrétées sont généralement initialement synthétisées sous forme d'une préprotéine comportant une "Séquence-signal" qui inclut le premier résidu. Cette séquence subit une excision protéolytique pendant ou immédiatement après le transport à travers la membrane [R. Scheckman, Trends Biochem (1985), 10, p.177]. Cependant ce système ne convient généralement pas dans le cas de protéines cytoplasmiques ou hétérologues du fait des problèmes de transport dûs soit à certaines parties de la séquence primaire de la protéine [J. Tommassen et coll, EMBO J. (1985), 4 p.1041] soit à une précipitation intra-cytoplasmique trop rapide de la protéine. Par ailleurs les mécanismes impliqués dans la sécrétion de protéines par les cellules encaryotes, telles que la SAH sécrétée par les cellules hépatiques, sont vraisemblablement assez différents des mécanismes de sécrétion mis en jeu dans des microorganismes tels que les bactéries gram-négatives [N.Wickner et H. Lodish, Science (1985), 230 p.400].

Il a été également proposé d'employer des digestions chimiques ou enzymatiques afin de convertir in vitro la protéine synthétisée par la bactérie sous la forme d'une protéine fusionnée. Cette conversion a pour but l'excision spécifique d'une séquence peptidique étrangère à la protéine désirée, située en position N-terminale et contenant la méthionine comme premier résidu. Un exemple simple est celui d'une protéine qui ne possède pas naturellement de résidus méthionine [R.E, Chance et coll., "Peptides : Synthèses-Structure-Fonction", D.H. Rich et E. Gross, ed., Pierce Chem. Co, Rocford, I11., (1981) p.721 et suivantes]. Dans ce cas, un traitement in vitro par le bromure de cyanogène permet l'excision de la méthionine N-terminale. Cependant ce cas ne se présente que très rarement dans le cas de protéines de poids moléculaire élevé.

Certaines protéases, comme la collagénase et le facteur X, reconnaissent une séquence de plusieurs acides aminés, ce qui les rend relativement spécifiques. [K. Nagai et H.C. Thogerson, Nature (1984), 309, p.810 et suivantes ; J. Gercino et D. Bastia, Proc. Natl. Acad. Sci. USA (1984), 81, p.4692 et suivantes]. Une construction génétique permet donc de positionner la séquence reconnue par la protéase en question devant le premier acide aminé de la protéine désirée. Cette protéine fusionnée devient ainsi un substrat de la protéase, le produit principal de la réaction étant la protéine possédant en position N-terminale le même acide aminé que la protéine mature. Cependant, l'inconvénient majeur de cette méthode réside dans le prix de la protéase surtout lorsqu'il s'agit de produire une protéine en grande quantité.

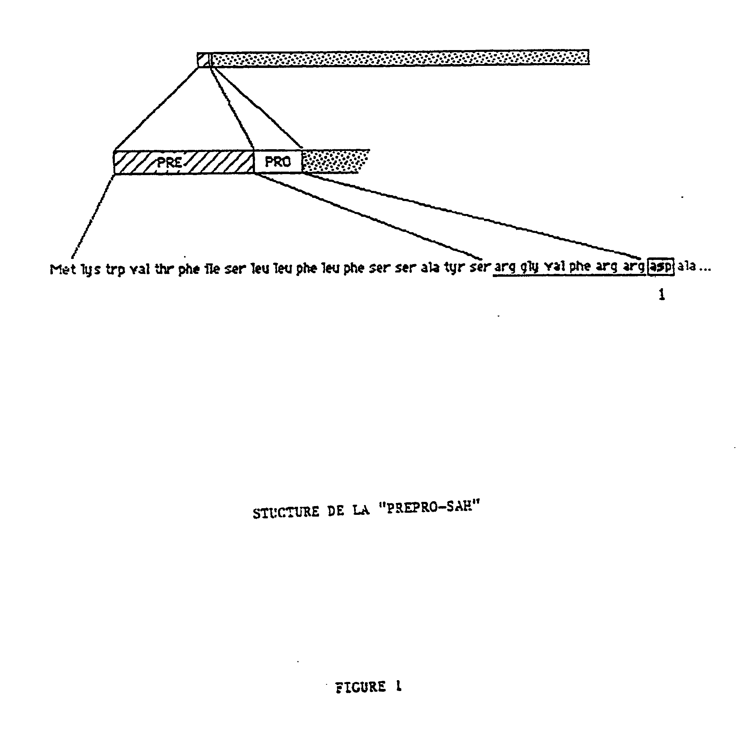

La SAH est synthétisée par les cellules humaines d'abord sous forme de prépro-SAH (figure 1). Une séquence signal de 18 acides aminés est enlevée pendant le transport de la SAH à travers le lumen du réticulum endoplasmique et il reste encore 6 acides aminés à l'extrémité N-terminale (Arg- Gly- Val- Phe- Arg- Arg-) qui ne sont pas présents dans la SAH circulante. Selon S.O. Brennan et R.W. Carrell, Biochim. Biophys. Acta (1980), 621, p.83 et suivantes, ce propeptide ne semble jouer aucun rôle dans la sécrétion de la SAH. Il est possible qu'une deuxième protéolyse spécifique s'effectue au niveau de l'appareil de Golgi ou dans la circulation sanguine, les deux résidus arginine formant le site de reconnaissance d'une protéase de spécificité analogue à celle de la trypsine. En effet, un variant, appelé "Albumine Christchurch", dû à une mutation qui transforme le dernier résidu arginine du propeptide en glutamine n'est pas converti in vivo en albumine mature mais est transformé in vitro en Glu-SAH en traitant le propeptide par une faible concentration de trypsine. Par ailleurs, la SAH mature sous forme native est résistante à la trypsine dans les même conditions [S.O. Brennan et coll., Biochim. Biophys. Acta, (1984) 802, p.24 et suivantes].

Il a maintenant été trouvé, et c'est ce qui fait l'objet de la présente invention, un procédé permettant de transformer en SAH mature une SAH produite par voie microbiologique sous forme de protéine fusionnée.

Le procédé selon la présente invention consiste :

– à modifier in vitro le gène de structure de la SAH de telle sorte qu'il possède 6 codons supplémentaires codant pour les 6 premiers acides aminés de la protéine cII du bactériophage lambda, puis à lier le gène de structure ainsi modifié à la séquence nucléotidique qui précéde naturellement le gène cII dans le gènome du bactériophage lambda et à un promoteur qui assure un niveau élevé de transcription,

– à produire, dans des conditions définies, au moyen d'une bactérie hôte contenant le gène modifié, une protéine hybride ("pseudo-pro-SAH") constituée par les 6 premiers acides aminés du gène cII suivis de la séquence de la SAH mature,

– à dénaturer et réduire puis renaturer la protéine hybride de façon à obtenir une protéine soluble dont la conformation est semblable à celle de la SAH d'origine naturelle, puis

– à modifier in vitro, au moyen de la trypsine, la protéine ainsi obtenue afin d'exciser le pseudo-pro-peptide et obtenir la SAH mature.

Il a également été trouvé que la SAH mature peut être obtenue en utilisant une extension peptidique N-terminale ("pseudo-pro-peptide") dont la séquence diffère de celle des 6 premiers acides aminés de la protéine cII du bactériophage lambda, à condition que cette extension permette une expression suffisante de la protéine fusionnée, présente l'hydrophilicité nécessaire et comporte un site de coupure par la trypsine. Par exemple, le "pseudo-pro-peptide" peut être constitué par les 5 premiers acides aminés de la séquence-signal de la pénicilline-amidase (6, si l'on compte le premier résidu méthionine).

Dans ce qui suit, la signification des termes techniques utilisés en biologie moléculaire est supposée connue (cf. par exemple J. Watson, "Biologie Moléculaire du Gène", édition française, Interéditions, 1978). Les méthodes couramment employées en biologie moléculaire du gène sont décrites, par exemple, par T. Maniatis et coll., Molecular Cloning, Cold Spring Harbor Laboratory Press, New-York, 1982). Dans ce qui suit seront décrits successivement la construction, les procédés d'expression du gène, la renaturation et la conversion par la trypsine de la "pseudo-pro-SAH".

A-CONSTRUCTION DU GENE "pseudo-pro-SAH".

1. Préparation d'ARN messager de foie

On utilise des cellules hépatiques, obtenues par exemple par biopsie, et on en extrait l'ARN messager selon la méthode décrite par exemple par V. Glisin et coll., Biochemistry (1974), 13, p. 2633 et suivantes ; et par R. Deeley et coll., J. Biol. Chem. (1977), 252, p. 8310 et suivantes. On traite la biopsie par une solution de thiocyanate de guanidine 6M, et l'on purifie l'ARN total par plusieurs cycles de précipitation dans l'éthanol à -20°C, centrifugation et redissolution des culots de centrifugation.

On enrichit la préparation en ARN messager par plusieurs cycles de chromatographie d'affinité sur des colonnes d'oligo (dT)-cellulose, selon la technique décrite par H. Aviv et P. Leder, Proc. Natl. Acad. Sci. (USA) (1972), 69, p. 1408 et suivantes. L'ARN messager ainsi isolé, contenant 1 à 2 % de l'ARN total, est conservé en solution aqueuse à -70°C.

On peut déterminer la proportion d'ARN messager spécifique de la sérum-albumine humaine au sein de la population totale (par exemple par traduction in vitro d'un aliquot de la solution d'ARN dans des lysats de réticulocytes de lapin). Une méthode consiste à utiliser le lysat de réticulocytes fournis par la société Amersham, suivant le protocole préconisé par ce fournisseur. On peut ainsi déterminer la fraction de protéine néoformée immunoprécipitable par des anticorps anti-albumine au sein de l'ensemble des protéines néoformées. On obtient par exemple une fraction de l'ordre de 6 %.

## 2. Synthèse de cDNA et clonage dans E.coli

### a. Synthèse du premier brin

A partir de la technique de G.N. Buell et coll., J. Biol. Chem. (1978), 253, p. 2471 et suivantes, modifiée, on utilise par exemple 5 µg d'ARN messager total dans un volume final de 50 microlitres d'une solution contenant : 100 mM Tris-HCl pH 8,3, 10 mM $MgCl_2$, 0,4 mM DTT, 20 mM KCl, 0,4 mM Na pyrophosphate, 1 mM de chaque nucléotide triphosphate (dNTP), 100 µg/ml de oligo$(dT)_{12-18}$, 0,5 U/ml d'inhibiteur de ribonucléases, 50 picomoles de traceur radioactif et 40 unités de Transcriptase réverse (Société Life Science, Inc.).

La réaction de transcription réverse de l'ARN messager en ADN complémentaire (ADNc) se poursuit pendant 1 heure à 42°C.

Le taux de synthèse de ADNc est calculé par mesure du taux d'incorporation du traceur radioactif en molécules acido-précipitables, selon une technique connue.

Après 1 heure, on arrête la réaction par addition d'EDTA (20 mM), et l'on détruit l'ARN messager par digestion alcaline dans 50 mM de NaOH, à 42°C, pendant 3 heures.

On sépare l'ADNc néoformé des dNTPs non-incorporés et des produits de dégradation alcaline des ARNs par chromatographie, par exemple, sur une colonne de Sephadex G100 (Pharmacia Fine Chemicals). On obtient 1,5 µg d'ADNc simple brin à partir de 5 µg d'ARN messager total.

### b. Synthèse du deuxième brin

L'ADNc simple brin est converti en ADN double brin par action du fragment "Klenow" de l'ADN polymérase I.

Les conditions de réaction sont : 100 mM Hepes pH 7, 10 mM $MgCl_2$, 2,5 mM DTT, 70 mM KCl, 0,5 mM de chaque dNTP, et 50 unités du fragment "Klenow" de l'ADN polymérase I (commercialisée par exemple par la Société New England Biolabs Inc.).

La réaction est poursuivie pendant 15 heures, à 15°C, et l'on sépare l'ADN double brin des dNTPs non incorporés à nouveau par chromatographie sur colonne de Sephadex G100.

### c. Clonage de l'ADN double brin

Pour supprimer les molécules d'ADN simple brin et obtenir un ADN double brin à extrémités franches, on traite les séquences non appariées par la nucléase $S_1$ selon la technique décrite par A. Efstradiatis et coll., Cell (1976), 7, p. 279 et suivantes. On sépare les ADNs néoforcés double brin selon leur taille par centrifugation dans un gradient de saccharose. On utilise généralement un gradient de 5 % - 20 % de saccharose en 50 mM Tris-HCl pH 8,5, 10 mM EDTA, 800 mM NaCl, centrifugé à 210000 g pendant 15 heures, à 20°C, et on effectue un fractionnement du gradient en aliquots après centrifugation.

On contrôle la taille des molécules dans chaque fraction par électrophorèse d'échantillons faite en parallèle avec des étalons d'ADN de tailles connues, et l'on regroupe les fractions contenant un ADN constitué par l'enchaînement de plus de 500 paires de bases.

Pour permettre le clonage de cet ADN on allonge d'abord ses extrémités 3' avec de l'oligo(dC), et on allonge parallèlement les extrémités 3' du site PstI du plasmide vecteur pBR322 avec de l'oligo(dG) selon la technique de F. Rougeon et coll., J. Biol. Chem. (1977), 252, p. 2209 et suivantes.

On hybride alors l'ADN double brin décrit ci-dessus au plasmide vecteur, selon par exemple la technique de L. Villa Komaroff et coll., Proc. Natl. Acad. Sci. (USA) (1978), 75, p. 3727 et suivantes.

On crée une "banque" de clones d'ADNcs de foie par transformation de la bactérie E.coli avec l'ADN ainsi décrit selon la méthode décrite par M. Mandel et A. Higa, J. Mol. Biol. (1970), 53, p. 154 et suivantes et M.

Dagert et S.D. Erlich., Gene (1979), 6, p. 23 et suivantes.

### d. Repérage des clones d'ADNc albumine

On utilise une technique d'hybridation sur colonies à l'aide d'oligonucléotides synthétiques dont les séquences sont déduites de la séquence protéique de l'albumine humaine (B. Meloun et coll., FEBS Letters (1975), 58, p. 134 et suivantes ; M. Grunstein et D. Hogness, Proc. Natl. Acad. Sci. (USA) (1975), 72, p. 3961 et suivantes ; R.B. Wallace et coll., Nucleic Acids Res. (1981), 9, p. 879 et suivantes).

Les clones sont cultivés par séries de 96 sur milieu de Luria contenant 25 µg/ml de tétracycline, en boîtes carrées, directement sur des filtres de nitrocellulose. Après croissance à 37°C puis amplification en présence de 250 µg/ml de chloramphénicol, les colonies sont lysées par la soude puis hybridées avec les oligonucléotides radioactivés en 5' par kination, dans une solution contenant : 5 X SSC, 0,5 % NP 40, 100 µg/ml ADN de sperme de saumon dénaturé par ébullition et refroidi rapidement dans la glace, 0,5 ng/ml d'oligonucléotide kinasé. L'hybridation est effectuée à 37°C pendant 18 heures. On lave ensuite les filtres en 5 X SSC, à 25°C, puis 37°C, puis 45°C et ce pendant quatre fois 15 minutes à chaque étape.

Les filtres sont alors exposés sur films Kodak X-OMAT, à -70°C, avec un écran amplificateur pendant 15 à 24 heures. Les clones hybridants avec les sondes sont réisolés puis lysés. L'ADN plasmidique est purifié par centrifugation en milieu chlorure de césium-bromure d'éthidium selon une technique connue.

On séquence l'ADN de l'insertion par la technique de Maxam-Gilbert (A. Maxam et W. Gilbert, Methods Enzymol. (1980), 65, p. 499 et suivantes) pour comparer la séquence protéique dérivée de la séquence nucléotidique et celle de la sérum-albumine humaine.

On identifie ainsi une série de clones dont les insertions correspondent à l'ensemble du gène de la sérum-albumine humaine.

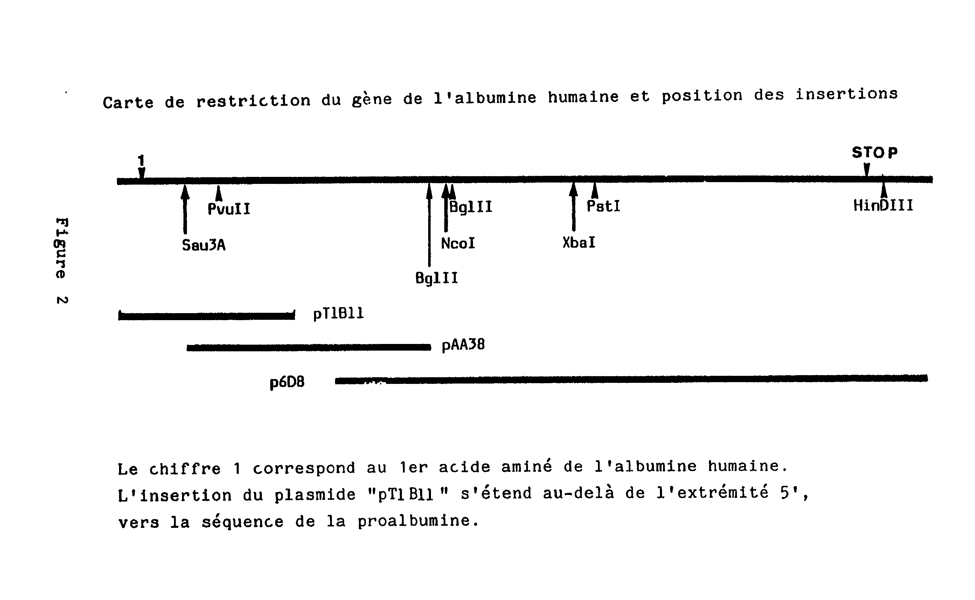

Dans la figure 2 est représentée la carte de restriction du gène de la sérum-albumine, ainsi que la position de trois des insertions les plus représentatives, désignées par "pT1B11", "pAA38", "p6D8".

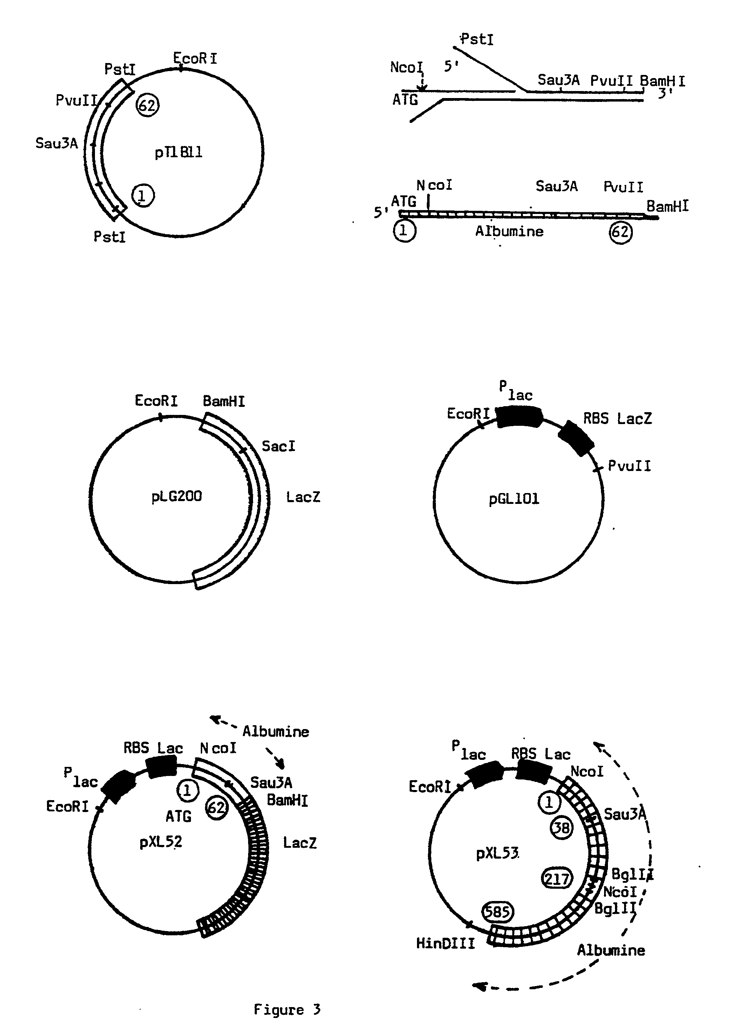

### e. Incorporation au grène de structure d'un codon d'initiation (figure 3)

a) On digère l'ADN du plasmide "pT1B11" par les enzymes PstI et PvuII, et on isole un fragment d'ADN de 125 paires de bases, correspondant à là séquence de l'extrémité 5' du gène de la sérum-albumine (acides aminés n° 1 à 62). On fixe à l'extrémité PvuII une séquence de jonction constituée du site de reconnaissance de l'enzyme BamHI. On obtient ainsi un fragment PstI-BamHI.

On prépare d'autre part un oligonucléotide synthétique ayant 21 bases de long, possédant un triplet "ATG" devant les nucléotides codant pour les acides aminés de la sérum-albumine humaine ainsi qu'un site de restriction NcoI, et dont la séquence est la suivante : 5'GAATCCATGGATGCACACAAG 3'.

On dénature le fragment d'ADN PstI-BamHI, et on l'hybride avec l'oligonucléotide synthétique. L'hybridation se fait par la séquence 5'...GATGCACACAAG 3', l'extrémité 3' du brin d'ADN complémentaire étant désappariée. On digère les extrémités désappariées, puis on polymérise dans le sens 5'...3' avec le fragment "Klenow" de l'ADN polymérase I, d'après les techniques de H. Jacobsen et coll., Eur. J. Biochem. (1974), 45, p. 623 et suivantes.

On obtient ainsi un fragment contenant en 5' une extrémité franche, un site NcoI puis le triplet ATG et en 3' un site BamHI.

b) On réalise la ligation de trois fragments d'ADN :

1) un fragment EcoRI-BamHI du plasmide "pLG200" (L. Guarenté et coll., Cell (1980) 20, p. 543 et suivantes) portant un gène de résistance aux antibiotiques, l'origine de réplication et l'extrécité 3' du gène de la β-galactosidase,

2) un fragment EcoRI-PvuII du plascide "pGL101" (G. Lauer et coll., J. Mol. Appl. Genet. (1981), 1, p. 139 et suivantes) portant le promoteur P$_{lac}$ et le site de fixation de ribosome (RBS) du gène lacZ d'E.coli,

3) le fragment d'ADN mutagénisé codant pour les 62 premiers acides aminés de l'albumine humaine.

On isole un plasmide (pXL52) qui réalise une fusion de l'extrémité 5' du gène de la sérum-albumine humaine avec le gène de la β-galactosidase d'E.coli.

### f. Construction du gène complet (figure 3)

On digère l'ADN du plasmide "p6D8" par EcoRI, et partiellement par BglII, selon une technique déjà décrite. On isole le grand fragment EcoRI-BglII contenant la séquence codant pour les 405 derniers acides aminés de la sérum-albumine humaine puis l'origine de replication du plasmide et le gène de résistance à la tétracycline.

On digère l'ADN du plasmide "pXL52" décrit ci-dessus par EcoRI et Sau3A, et on isole un fragment contenant 200 paires de bases.

On digère l'ADN du plasmide "pAA38" par Sau3A et on isole un fragment contenant 540 paires de bases.

On ligature les trois fragments (dans l'ordre [pXL52 EcoRI-Sau3A] - [pAA38-Sau3A] - [p6D8 BglII-EcoRI]) en tirant profit de la compatibilité entre les sites Sau3A et BglII. On obtient un plasmide appelé "pXL53", dont la qualité de la construction est contrôlée par un séquençage complet du fragment compris entre le site EcoRI et le site PstI correspondant à la jonction de l'insertion et du plasmide vecteur.

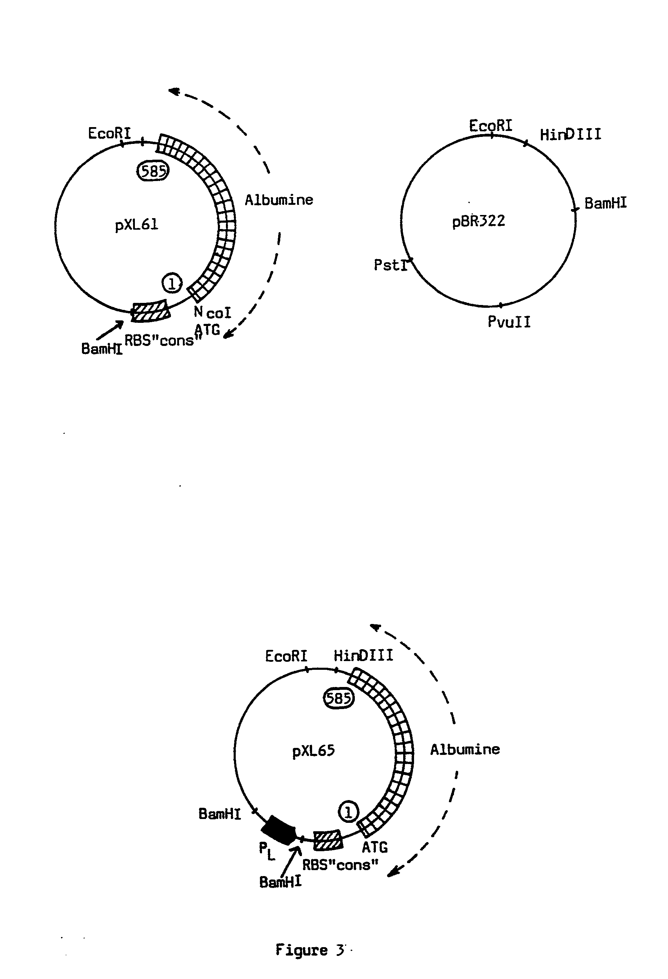

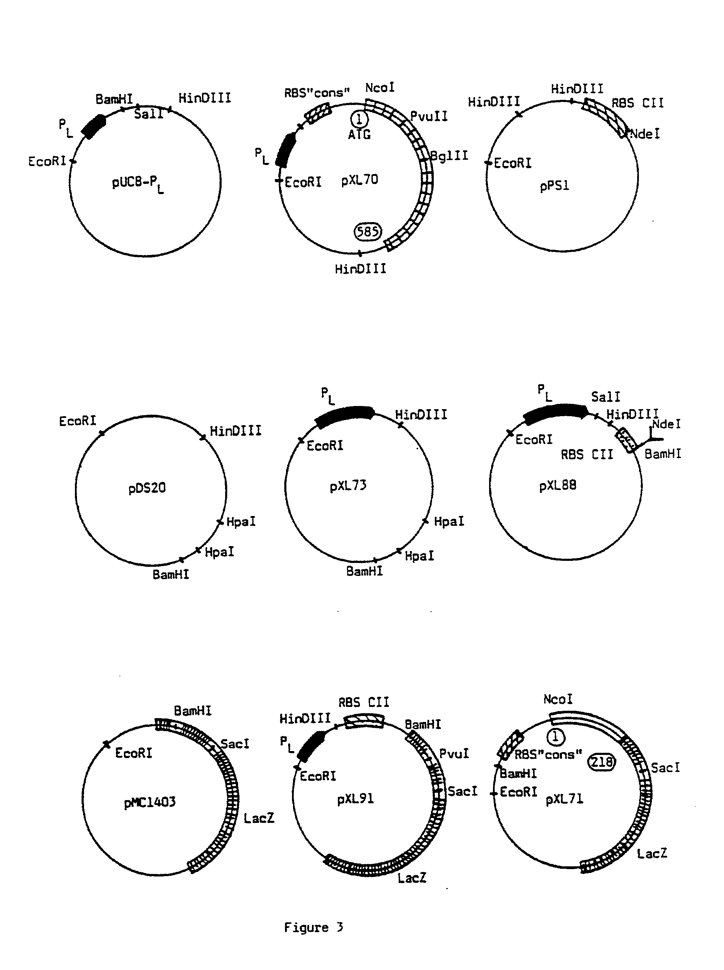

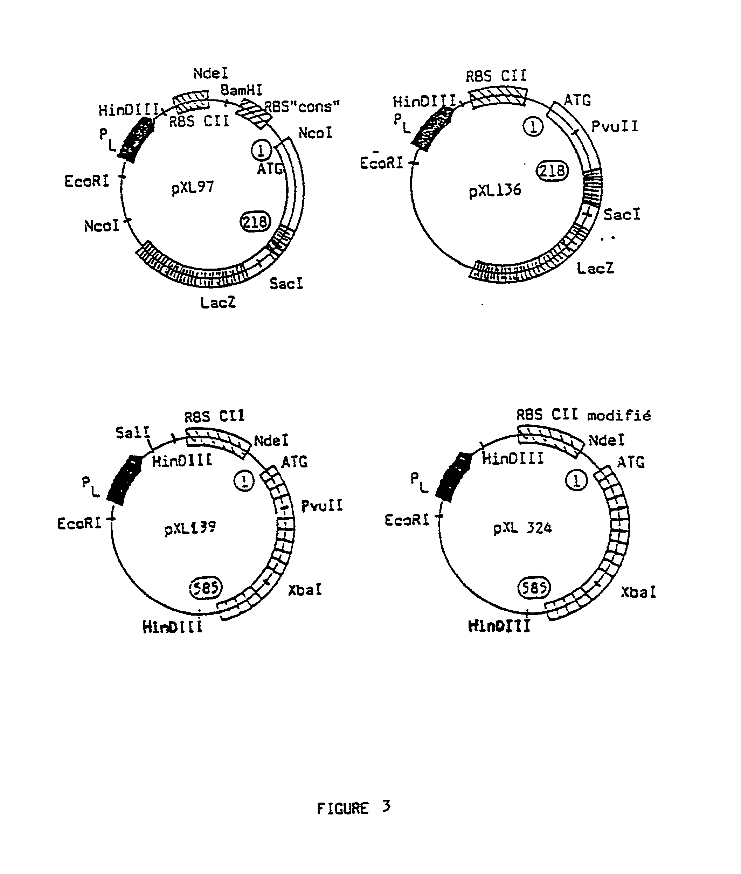

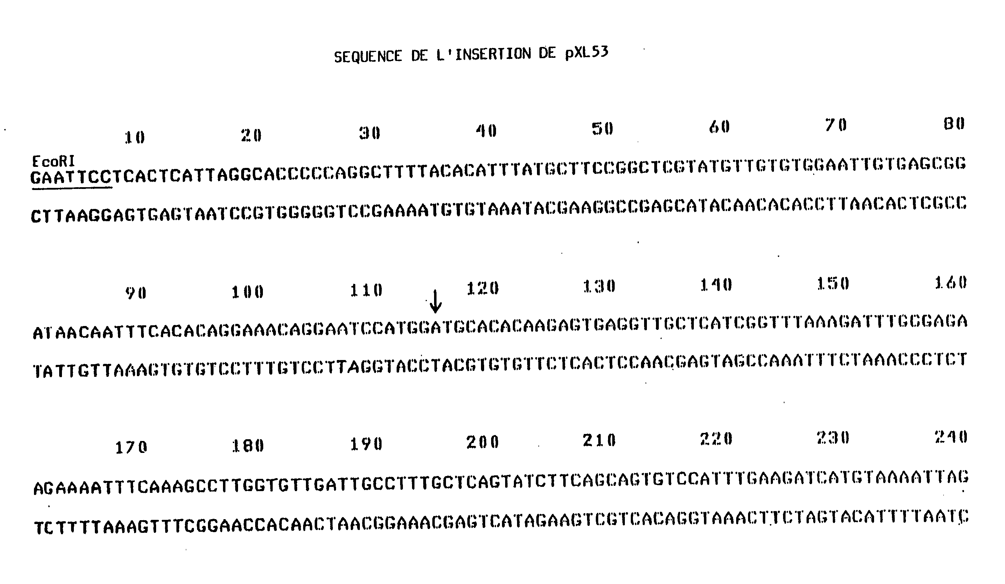

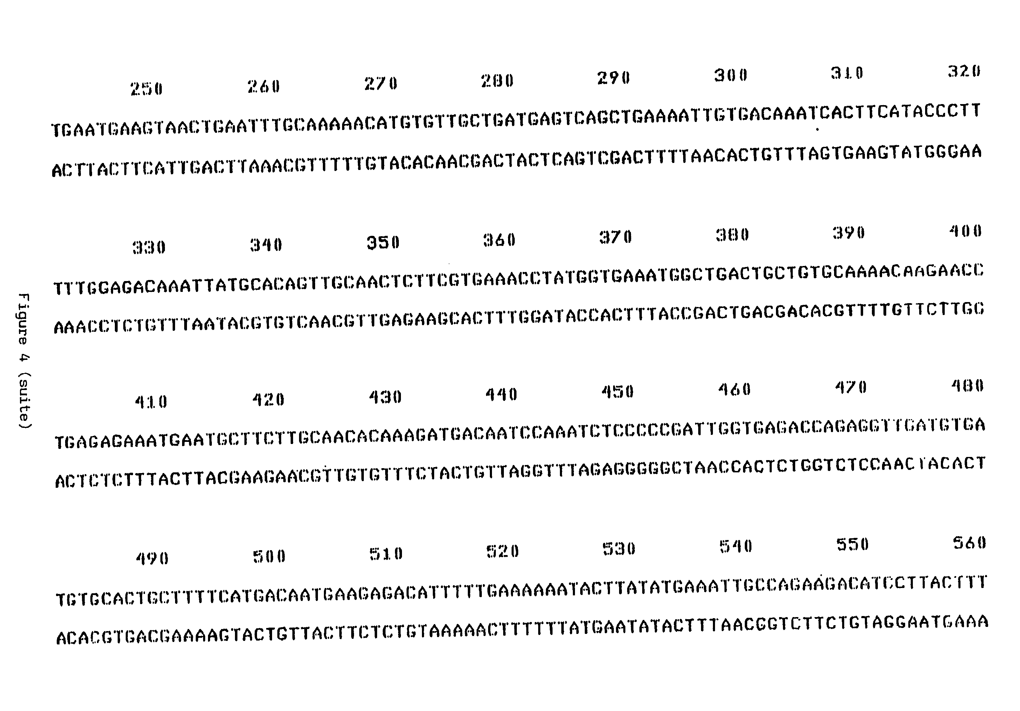

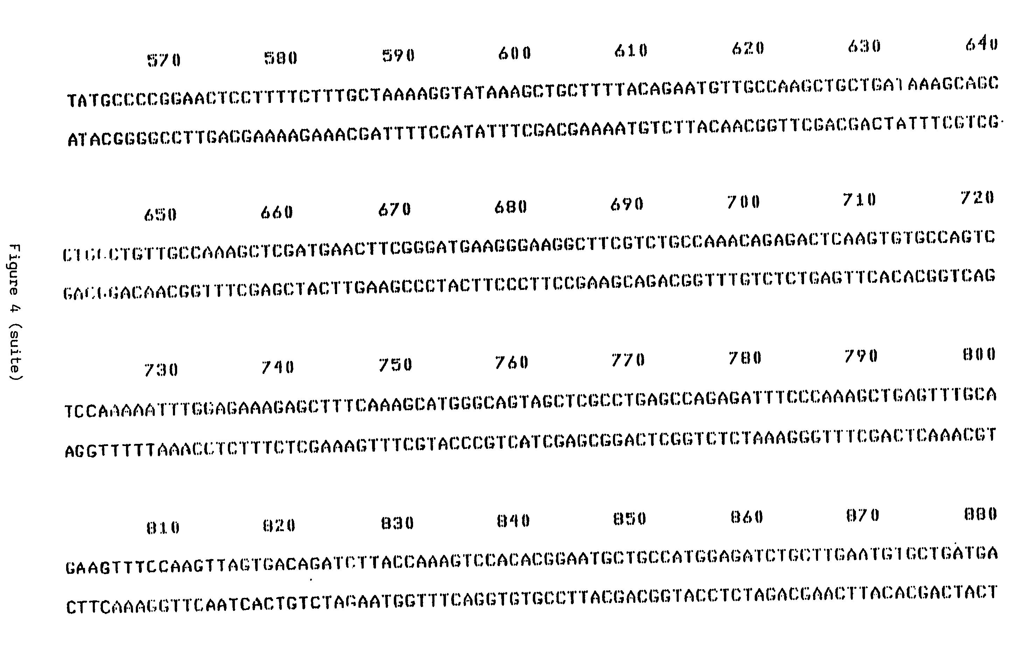

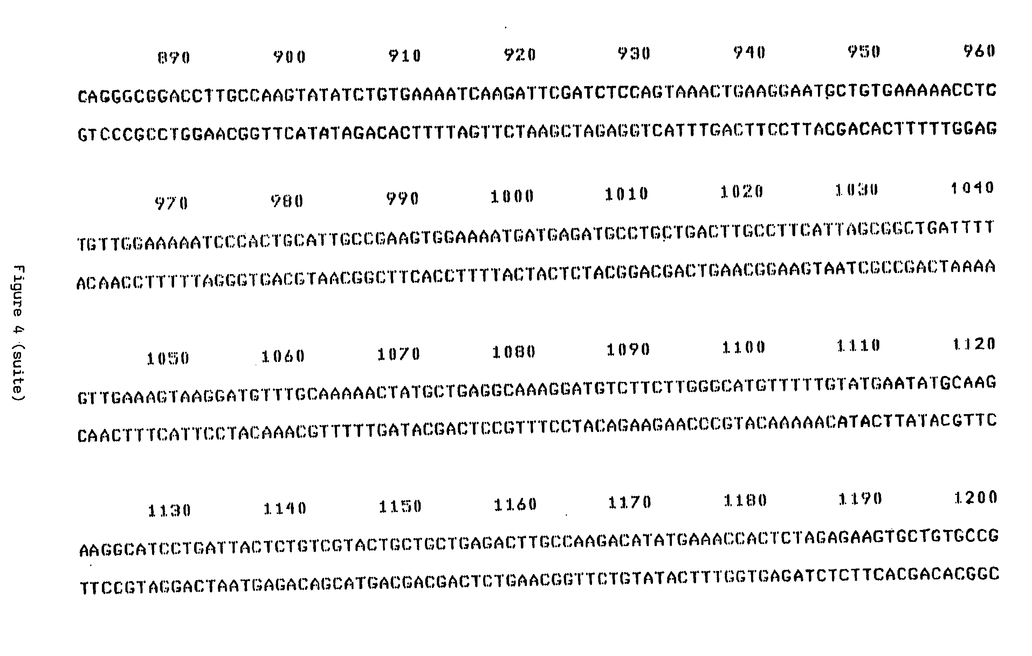

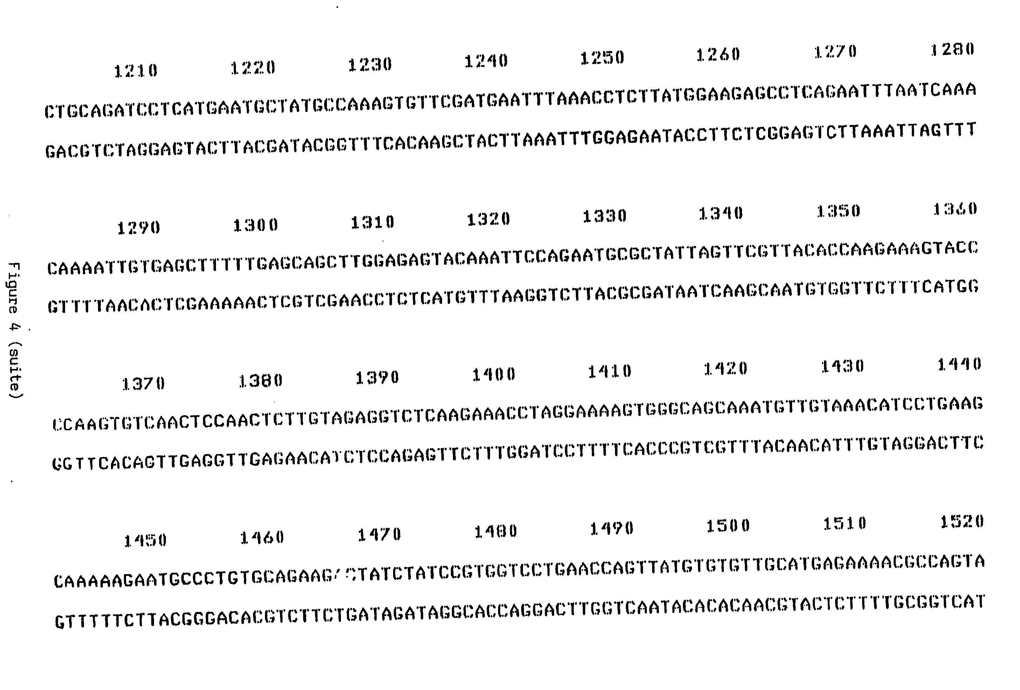

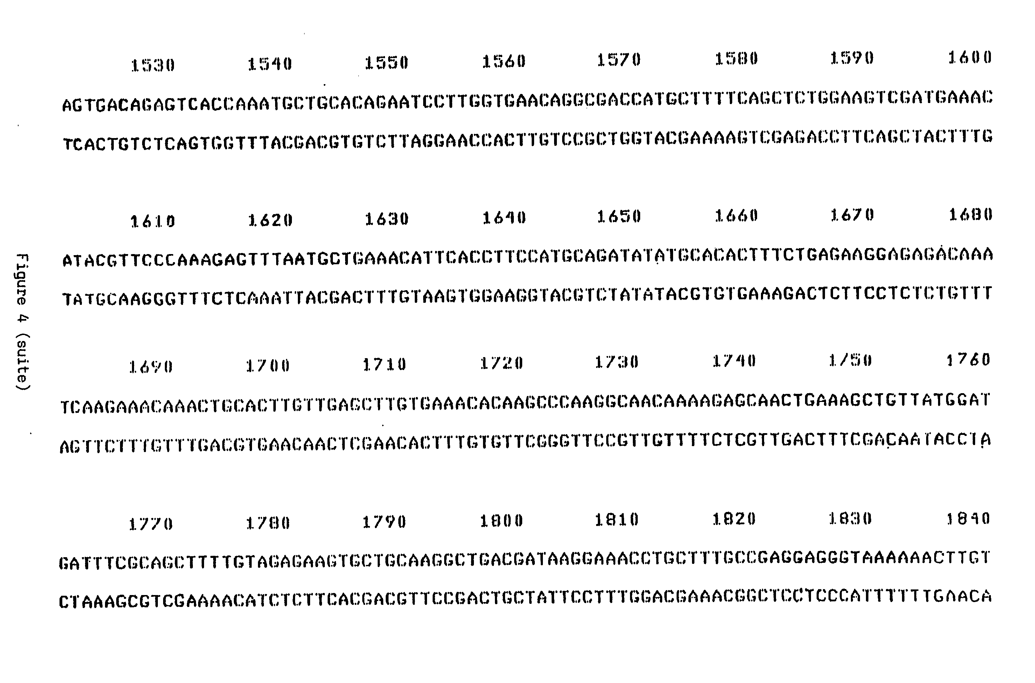

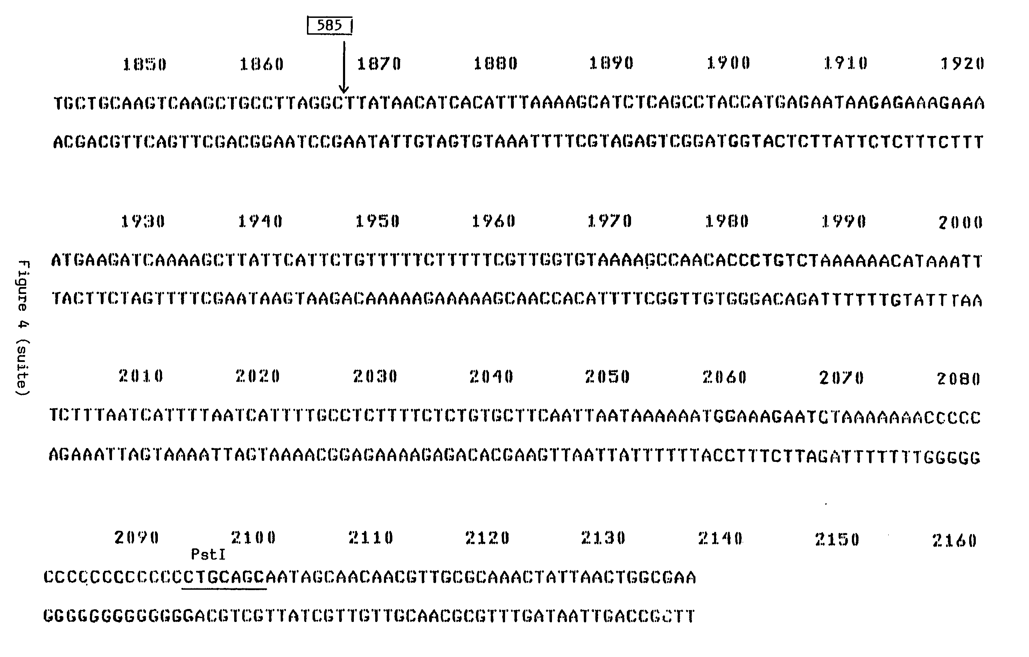

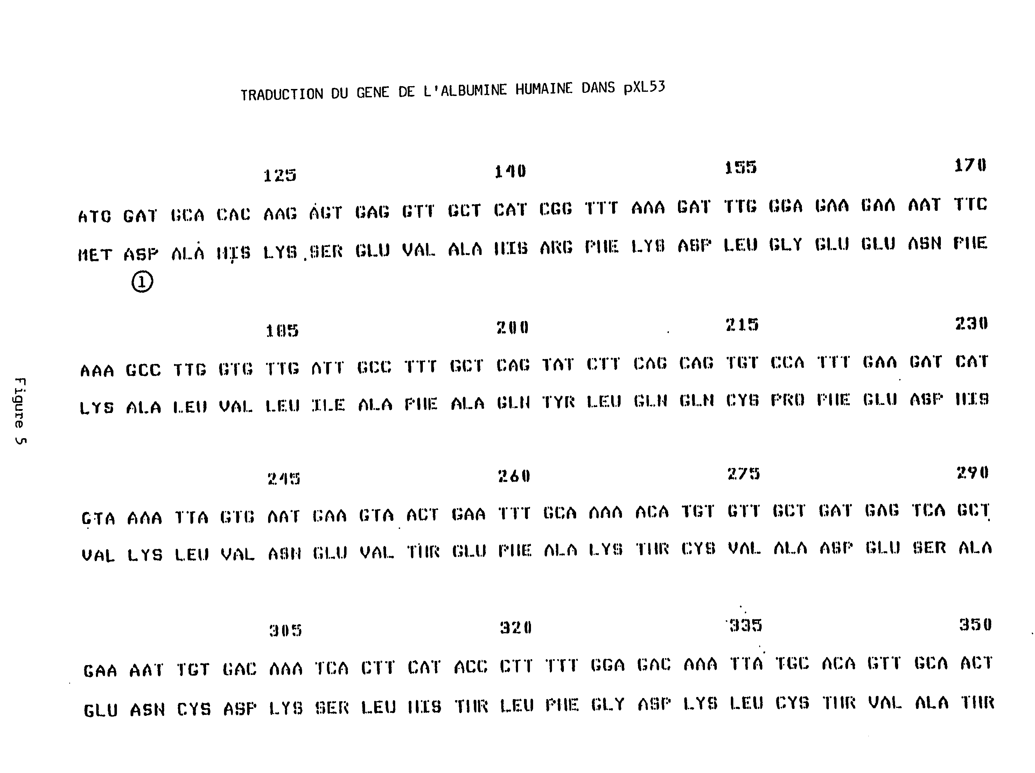

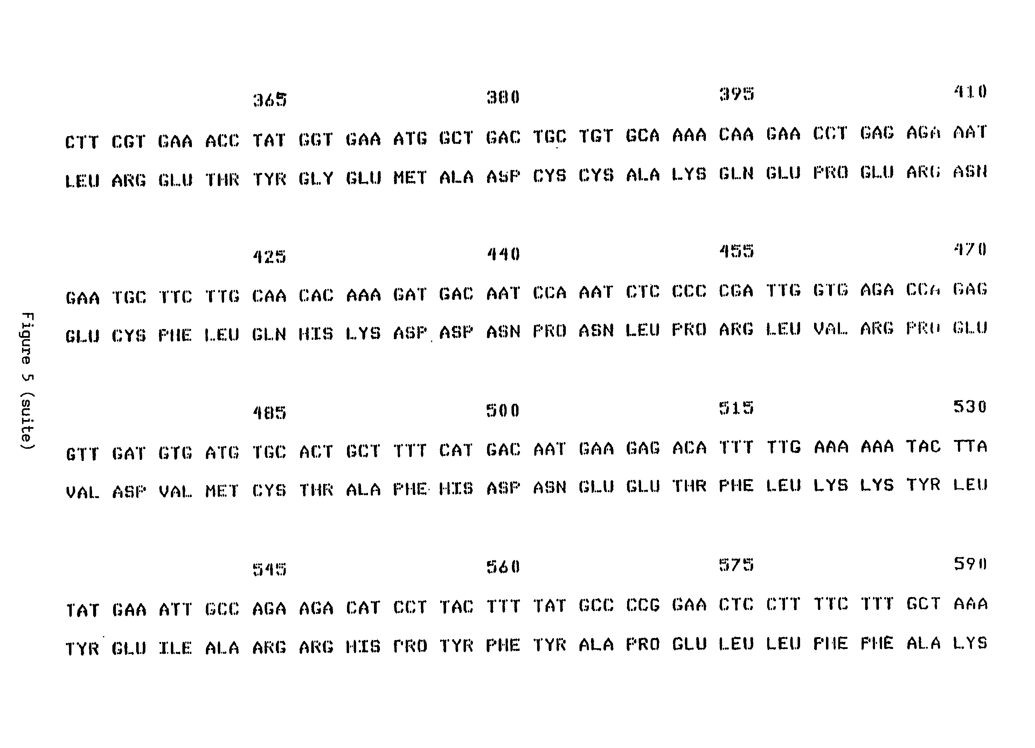

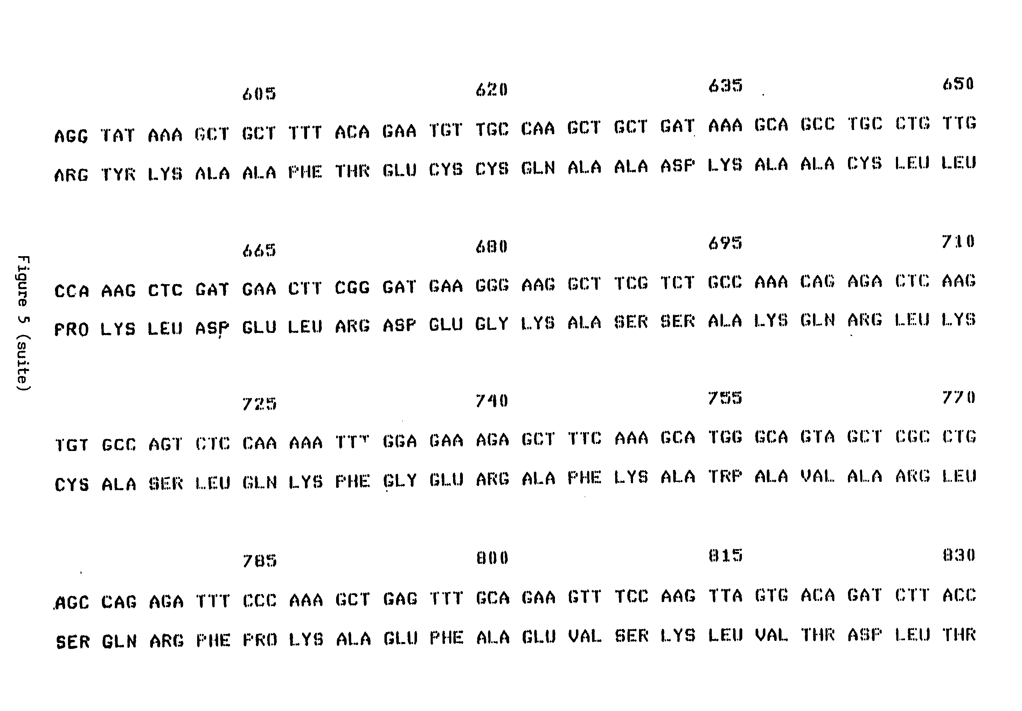

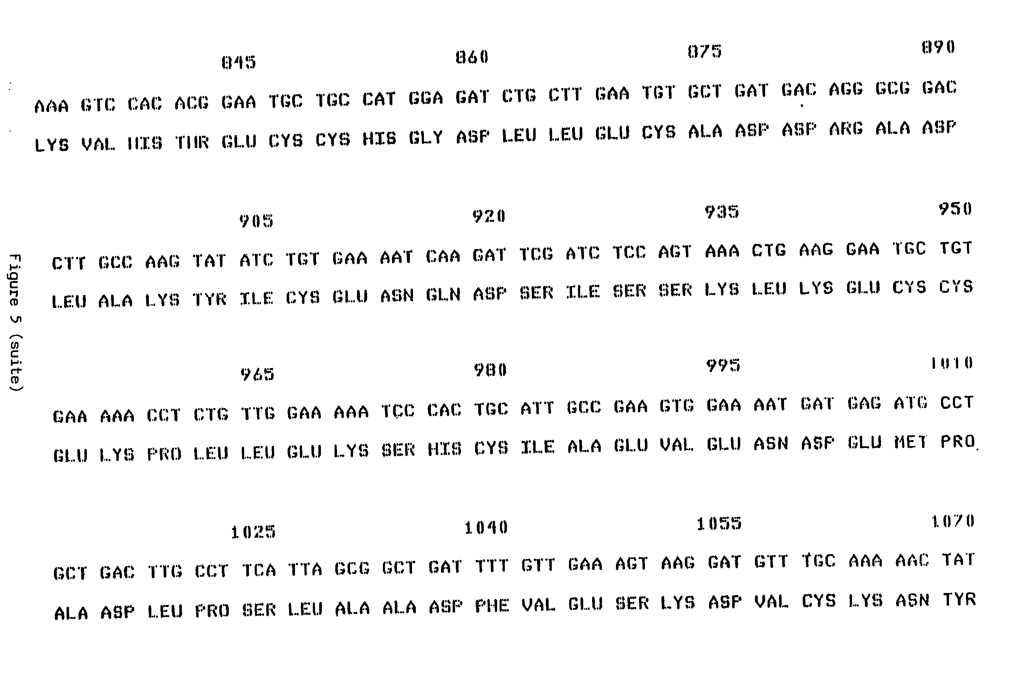

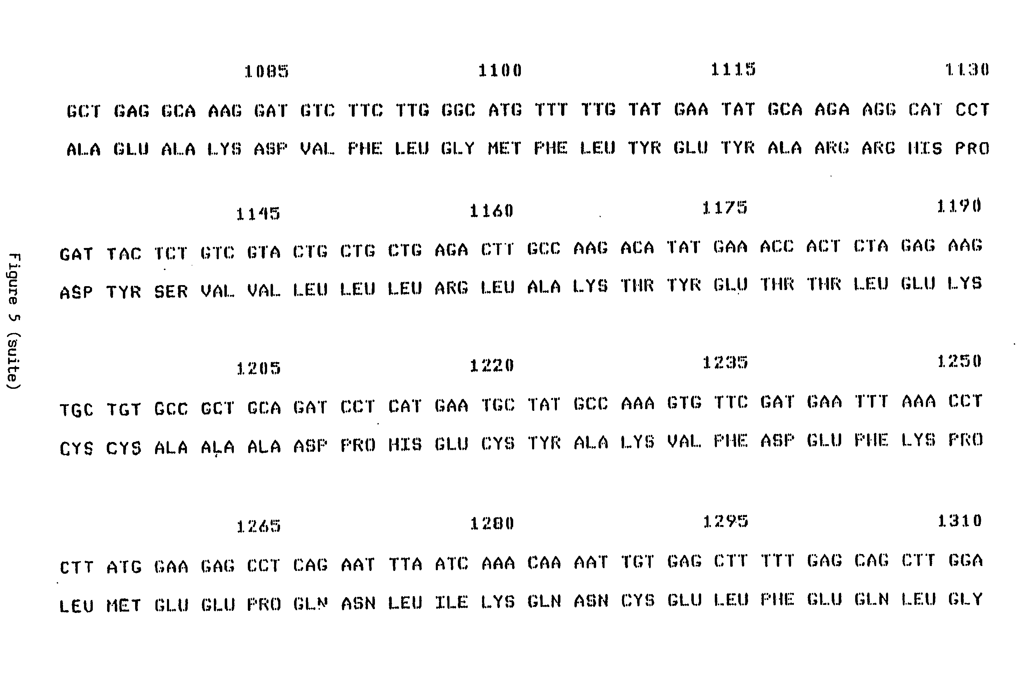

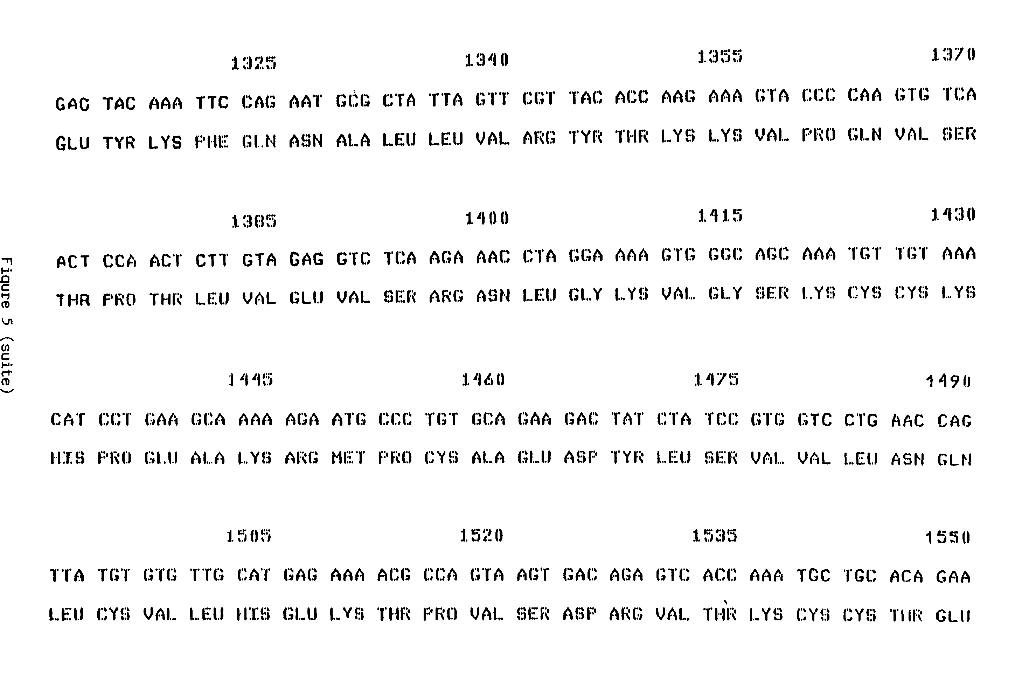

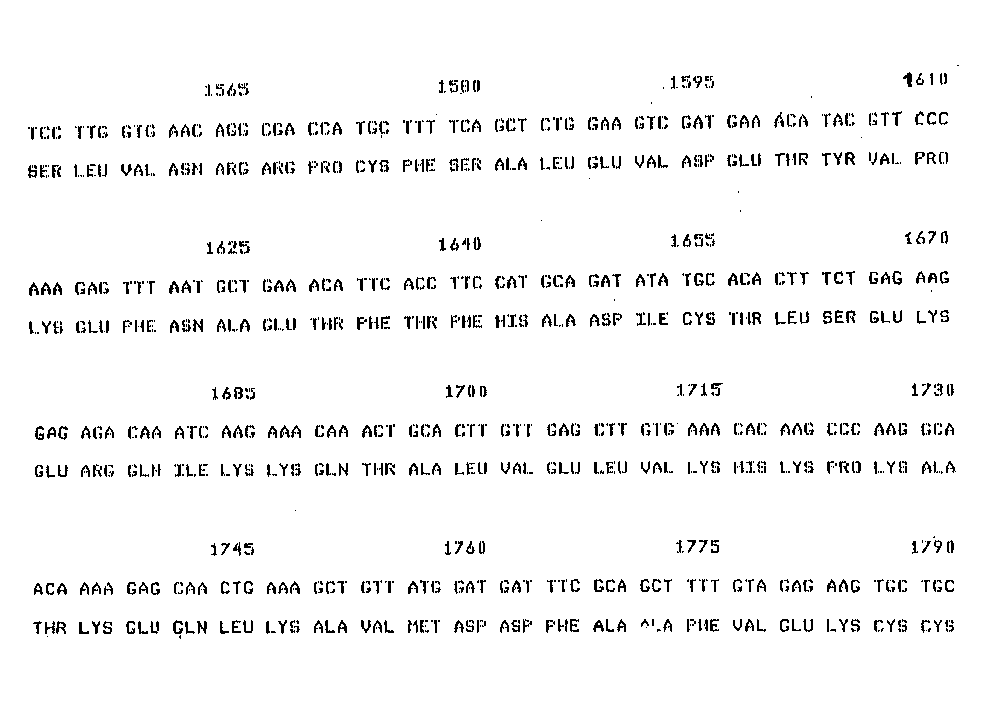

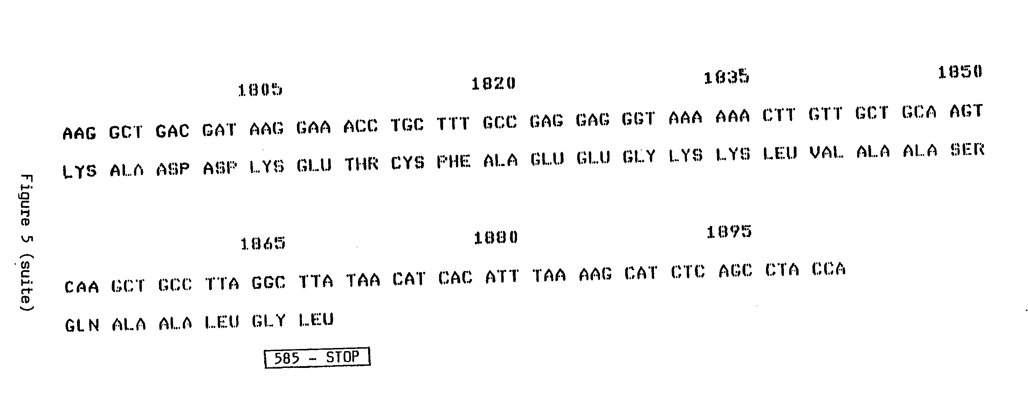

La séquence nucléotidique complète, ainsi que la séquence protéique dérivée, sont représentées dans les figures 4 et 5.

Les variations observées entre cette séquence et la séquence protéique publiée (B. Meloun et coll, FEBS Letters (1975), 58, p. 134 et suivantes ; M. Dayhoff, Atlas of Protein sequence and structure (1978), 5, supplément 3, p. 306) sont les suivantes :

| Position | Meloun et coll. | Sérum-albumine humaine déduite de la séquence de "pXL53" |
|---|---|---|
| 131 | Glutamine | Acide glutamique |
| 364 | Histidine | Alanine |
| 367 | Tyrosine | Histidine |
| 370 | Alanine | Tyrosine |
| 381 | Valine | Méthionine |
| 464 | Acide glutamique | Histidine |
| 465 | Histidine | Acide glutamique |
| 501 | Glutamine | Acide glutamique |

3. Construction de systèmes d'expression de la méthionyl-sérum-albumine humaine

a. Utilisation du promoteur "P$_L$" du bactériographe lambda

On linéarise le plasmide "pXL53" par digestion partielle par l'enzyme NcoI, en ne considérant que le site NcoI en 5' du codon d'initiation et on forme des bords francs par remplissage selon la technique de R.M. Wartell et W.S. Reznikoff, Gene (1980), 9, p. 307 et suivantes).

On synthétise un "adaptateur" contenant en 5' une séquence correspondant au site de reconnaissance d'une enzyme de restriction telle que BamHI, puis une séquence correspondant à un site de fixation de ribosomes (RBS "consensus" ou "théorique"). La séquence de l'adaptateur est : 5'GGATCCTAGGAGGAAC 3'.

La ligation de l'adaptateur en 5' d'un ADN à bords francs a été décrite, par exemple, par C.P. Bahl et coll., Gene (1976), 1, p. 81 et suivantes.

La méthode consiste à effectuer la réaction sur 20 microlitres d'une solution contenant 50 mM Tris, HCl pH = 7,5, 10 mM MgCl$_2$, 15 mM DTT, 1mM ATP, 50 µg/ml d'adaptateur, 20 µg/ml d'ADN et 1 unité d'ADN-ligase (New England Biolabs Inc.). La réaction est poursuivie pendant 10 heures à 15°C. Cette ligation crée un site BamHI sans supprimer le site NcoI.

On digère le produit de ligation par BamHI et par HinDIII. Du fait de la présence d'un site HinDIII en 3' du gène de la sérum-albumine humaine, on obtient un fragment d'ADN contenant la totalité de la séquence codante.

On sous-clone le fragment HinDIII-BamHI ainsi obtenu par exemple dans le plasmide "pBR322" en transformant E.coli selon la méthode déjà décrite ci-dessus pour obtenir le plasmide "pXL61".

Le plasmide "pXL61" ne contient pas de promoteur.

Le promoteur "P$_L$" du bactériophage lambda est placé sur le chromosome du bactériophage entre un site BglII et un site BamHI (voir E. Szybalski et W. Szybalski, Gene (1979) 7, p. 217 et suivantes), et dont la séquence nucléotidique est connue (F. Sanger et coll., J. Mol. Biol. (1982), 162, p. 279 et suivantes). On peut cloner ce fragment et modifier ses sites de restriction selon des méthodes connues.

On note que les plasmides portant P$_L$ doivent être propagés dans des souches de E.coli portant le gène répresseur cI, ceci afin d'éviter que ce promoteur ne s'exprime de façon constitutive.

Dans une première construction, P$_L$ est disponible sous forme d'un fragment BamHI à partir du plasmide "pPL-lambda" (Pharmacia P.L. Biochemicals). L'insertion de ce fragment BamHI dans le site BamHI du plasmide "pXL61" permet d'obtenir le plasmide "pXL65", dans lequel on a vérifié que l'orientation du promoteur par rapport au gène de structure de la sérum-albumine humaine est correcte.

D'autres constructions peuvent être réalisées à partir de plasmides disponibles. On peut, par exemple, exciser du plasmide "pP$_L$-lambda" un fragment HaeIII-HaeIII contenant le promoteur P$_L$ et l'insérer dans le site SmaI d'une séquence de clonage multisites portée sur un plasmide, tel que le plasmide "pUC8" (J. Vieira et J. Messing, Gene, (1982), 79, p. 259 et suivantes) pour obtenir "pUC8-P$_L$"" dans lequel le site EcoRI est en 5' du promoteur.

A partir du plasmide "pPS1" (P. Sarmientos et coll., Cell (1983), 32, p. 1337 et suivantes), on peut d'abord détruire le site HinDIII le plus proche du site NdeI (figure 3) puis remplacer le petit fragment EcoRI-HinDIII par, d'une part, le fragment EcoRI-BamHI du plasmide "pUC8-P$_L$" contenant le promoteur P$_L$, et, d'autre part, le fragment BamHI-HinDIII du plasmide "pXL61" contenant le gène de la sérum-albumine. On obtient ainsi le plasmide "pXL70" dans lequel l'ensemble P$_L$-RBS "consensus "-ATG-gène de la sérum-albumine humaine est porté sur un fragment d'ADN EcoRI-HinDIII.

### b. Remplacement du RBS "consensus" par celui du gène cII du bactériophage lambda

Le gène cII du bactériophage lambda, dont la séquence et le site d'initiation sont connus, peut être traduit avec efficacité (E. Schwarz et coll., Nature (1978), 272, p. 410 et suivantes).

On construit un plasmide contenant le système d'expression "Promoteur "P$_L$" - RBS cII - ATG - gène sérum-albumine".

Par exemple, on peut après avoir détruit le site BamHI de "pUC8-P$_L$" par action de l'enzyme SI (A.J. Berk et P.A. Sharp, Cell (1977), 12, p. 72) isoler un fragment EcoRI-HinDIII contenant le promoteur P$_L$ et ensuite lier ce fragment avec le grand fragment EcoRI-HinDIII du plasmide "pDS20" (G. Duester et coll., Cell (1982), 30, p. 855 et suivantes), pour obtenir le plasmide "pXL73".

Le RBS du gène cII est extrait du plasmide "pPS1". On digère ce plasmide par NdeI et on insère un adaptateur BamHI après formation d'extrémités franches. On excise alors le RBS sous forme d'un fragment Hin-DIII-BamHI.

On construit d'abord un plasmide "pXL88" dans lequel ce fragment HinDIII-BamHI est lié au grand fragment HinDIII-BamHI du plasmide "pXL73". Dans le nouveau plasmide "pXL88", le RBS cII est inséré dans la bonne orientation par rapport au promoteur P$_L$, le tout dans un système multisites de telle sorte que l'ensemble P$_L$-RBS cII soit porté sur un fragment d'ADN EcoRI-BamHI de 578 paires de bases.

Le fragment EcoRI-BamHI de 578 paires de bases est sous-cloné entre les sites EcoRI et BamHI du plasmide "pMC1403" (M.J. Casadaban et coll., J. Bacteriol. (1980), 143, p. 971 et suivantes) qui porte le gène de la β-galactosidase (lacZ) après le site BamHI. Cette construction conduit au plasmide "pXL91" dans lequel le gène de la β-galactosidase est exprimé sous contrôle du système "P$_L$-RBS cII".

On sous-clone le fragment BamHI-BglII du plasmide "pXL61" décrit précédemment dans le site BamHI du plasmide "pMC1403". (La ligation d'un site BglII dans un site BamHI est possible, mais l'excision par BamHI en BglII ne l'est plus ; il ne reste donc qu'un site BamHI).

Cette construction ("pXL71") aboutit à l'insertion d'un fragment d'ADN de 700 paires de bases comportant la séquence "BamHI-[RBS "consensus"-ATG-NcoI-gène partiel de la sérum-albumine -(codant pour les acides aminés 1 à 218)-gène de la β-galactosidase].

On coupe ce plasmide par BamHI et SacI (le site SacI est présent dans le gène de la β-galactosidase) et on l'insère dans le plasmide "pXL91" décrit précédemment à la place du fragment préexistant BamHI-SacI.

On aboutit alors au plasmide "pXL97" dont l'insertion a la structure suivante : "Site EcoRI - P$_L$ - RBS cII - site BamHI-RBS "consensus"- site NcoI - ATG - gène partiel de la sérum-albumine -gène de la β-galactosidase".

On digère le plasmide "pXL97" par BamHI et partiellement par NcoI en ne considérant que le site NcoI proche du codon d'initiation et on forme les bords francs par action de la nucléase SI, puis on le referme sur lui-même. Cette manipulation, d'une part, supprime la séquence d'ADN du RBS "consensus" et, d'autre part, met en phase un ATG du RBS cII avec la séquence de la sérum-albumine.

On obtient ainsi le plasmide "pXL136" qui comporte la séquence "site EcoRI-P$_L$-RBS cII-ATG-gène partiel de la sérum-albumine-gène de la β-galactosidase".

Le gène partiel de la sérum-albumine possédant un site PvuII, on digère le plasmide "pXL136" par EcoRI et PvuII et on extrait un fragment de 760 paires de bases qui est inséré entre les sites EcoRI et PvuII du plasmide "pXL70" décrit précédemment. On obtient ainsi le plasmide "pXL139" qui porte la structure "P$_L$-RBS cII-gène sérum-albumine complet" sur un fragment EcoRI-HinDIII, comme le plasmide "pXL70" et qui porte la substitution RBS "consensus" par celui du gène cII.

On coupe le plasmide "pXL139" décrit précédemment au site unique SalI, entre le promoteur PL et le RBS cII. On digère l'ADN par l'enzyme Ba131, de telle sorte que le site de fin de transcription tR1 en 5′ du RBS cII soit digéré puis on ajoute un adaptateur HinDIII et on isole le fragment HinDIII-XbaI contenant le RBS cII amputé de tR1 et les 357 premiers codons du gène de la sérum-albumine humaine. On combine ce fragment HinDIII-XbaI avec d'une part le fragment XbaI-EcoRI du plasmide pXL139 contenant la fin du gène de la sérum-albumine humaine et d'autre part le fragment EcoRI-HinDIII portant le promoteur $P_L$ obtenu à partir du plasmide pUC8-$P_L$ après destruction du site BamHI. On obtient ainsi le plasmide pXL324.

### 4. Construction d'un plasmide d'expression pour la "pseudo-pro-SAH"

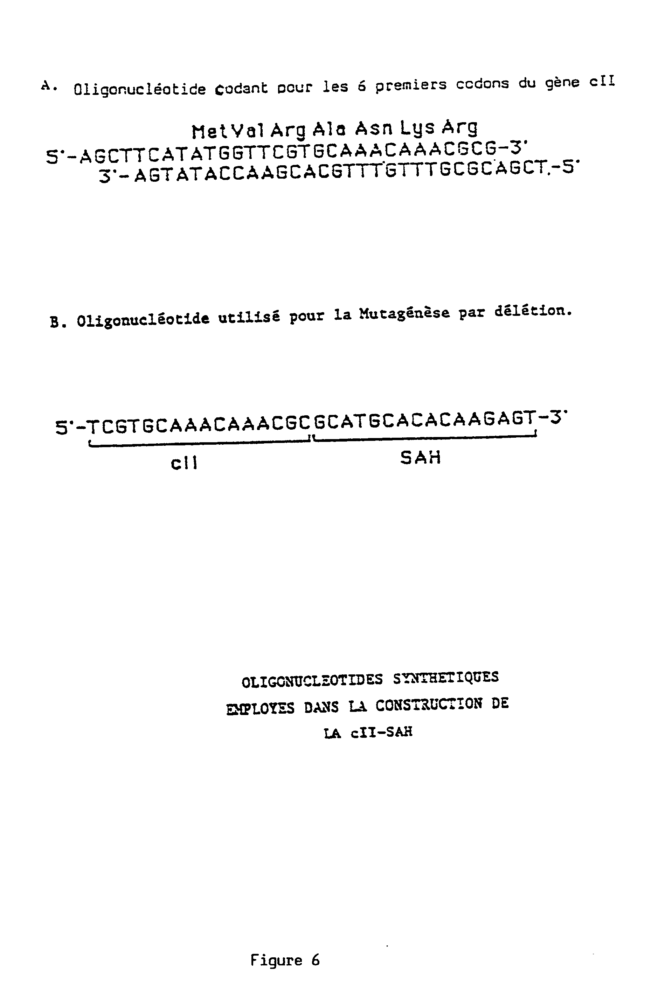

Un fragment d'ADN est construit par hybridation de deux oligonucléotides synthétiques ayant la structure donnée dans la figure 6A. La séquence contient un codon de démarrage "ATG" suivi par les 6 premiers codons du gène cII du bactériophage lambda. Ce fragment possède une extrémité cohésive de type HinDIII et une autre extrémité cohésive de type SalI. Ce fragment synthétique est cloné entre les sites HinDIII et SalI du vecteur M13mp10 (J. Messing, Methods Enzymol., (1984), 101, p.20 et suivantes). Le DNA en forme réplicative purifié à partir de cellules infectées par le bactériophage résultant est utilisé dans l'étape suivante de construction.

Un fragment SalI-BglII de 765 paires de bases provenant du plasmide pXL324 contenant le début du gène (ADNc) codant pour la SAH est cloné dans ce bactériophage recombinant. La souche de E.coli JM101 est infectée par ce nouveau bactériophage et le surnageant d'une culture de 5 heures est utilisé comme source de particules phagiques contenant l'ADN simple brin caractéristique des phages filamenteux de type M13. Ce simple brin sert ensuite de matrice pour une mutagénèse dirigée par oligonucléotide permettant de supprimer la séquence comprise entre le sixième codon du gène cII et le premier codon de la SAH mature (GAT) selon les méthodes décrites, par exemple, par J.P. Adelman et coll., DNA (1983), 2, p.183. L'oligonucléotide utilisé dans cette mutagénèse dirigée est décrit dans la figure 6B. Le phage résultant contient le début d'un nouveau gène fusionné. La structure de fragment d'ADN utilisé dans les constructions ultérieures est vérifiée par la méthode de séquençage enzymatique (F. Sanger et coll., Proc. Natl. Acad. Sci. USA, (1977), 74, p.5463).

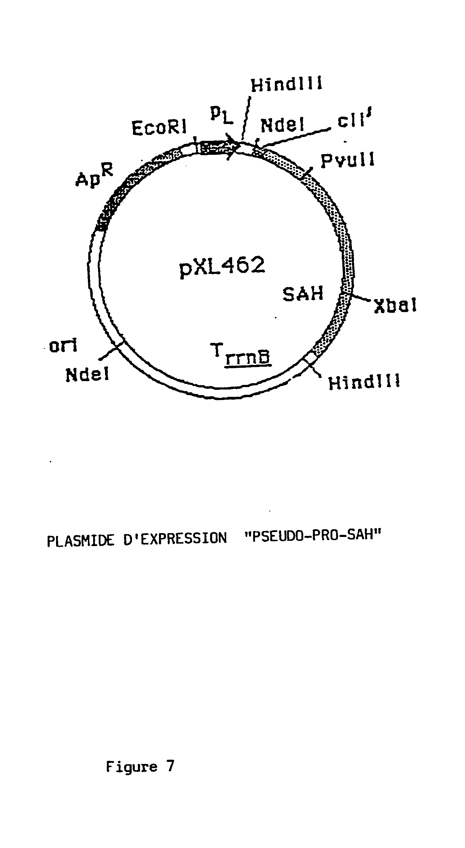

Une reconstruction du gène complet codant pour la fusion "pseudo-pro-SAH" est ensuite effectuée. Un vecteur contenant un gène de résistance à l'ampicilline, une origine de réplication, un terminateur de transcription et une partie de l'ADNc codant pour la SAH est préparé à partir du plasmide pXL70 en traitant ce plasmide par les enzymes de restriction EcoRI et PvuII. Le fragment de 7200 paires de bases environ est purifié par électrophorèse en gel d'agarose et électroélution. Un fragment de 430 paires de bases contenant le promoteur $P_L$ et le site d'accrochage sur le ribosome (RBS) modifié du gène cII est purifié à partir d'une digestion du plasmide "pXL324" par les enzymes EcoRI et NdeI par électrophorèse en gel de polyacrylamide et électroélution. Un fragment NdeI-PvuII de 200 paires de bases contenant le début du gène hybride cII-SAH est purifié à partir de la forme réplicative du bactériophage M13 recombiné modifié par mutagénèse in vitro décrite ci-dessus. Une réaction de ligation à trois partenaires a été effectuée. Le plasmide résultant est appelé "pXL462" (figure 7).

Le plasmide "pXL462" a été introduit dans la souche G819 par transformation. Cette souche est dérivée de la souche E103S (L. SIMON, Waksman Institute for Microbiology, Rutgers-The State University, Piscataway, N.J.,USA) par transformation avec le plasmide pRK248cIts (H-U. Bernard et coll., Gene (1979), p.59 et suivantes). Ce plasmide est compatible avec "pXL462" et porte le gène cI du bactériophage lambda qui code pour un répresseur thermosensible du promoteur $P_L$. Ce répresseur devient en effet inactif au-dessus de 38,5°C. La souche obtenue porte le numéro G1398.

A partir du plasmide pXL462, d'autres plasmides ont été construits où le promoteur $P_L$ contenu sur un fragment de restriction EcoRI-HinDIII a été remplacé par différents promoteurs bactériens inductibles. La construction de ces plasmides a utilisé le site XbaI unique de pXL462 et une réaction de ligation à trois partenaires du type de celle décrite ci-dessus (voir figure 7). La présente invention ne dépendant pas du type de promoteur bactérien utilisé, seul le cas du plasmide pXL462 portant le promoteur $P_L$ sera évoqué dans ce qui suit.

### B. PRODUCTION DE cII-SAH PAR VOIE MICROBIOLOGIQUE

### 1. Culture et Induction

A partir d'un réisolaient de la souche G1398 sur une boîte de Pétri gélosée à base de milieu LB contenant 50 microgrammes/ml d'ampicilline (LBAp) préalablement incubée à 30°C, une préculture est diluée 100 fois dans le même milieu et la culture est incubée à 30°C avec agitation. Lorsque la densité optique lue à 610 nanomètres atteint 1,0 la culture est alors portée à 42°C pendant 90 minutes avec agitation.

## 2. Sonication, récupération de la cII-SAH

Le culot cellulaire collecté par centrifugation est resuspendu dans 1/30 de volumes de PBS (0,2 g/l KCl, 0,2 g/l $KH_2PO_4$, 8 g/l NaCl et 1,25 g/l $Na_2HPO_4$). Après incubation pendant 15 minutes à une température voisine de 20°C en présence de lysozyme de blanc d'oeuf à 1 mg/ml, la sonication des bactéries est effectuée à 0°C, par exemple, avec un sonicateur Branson (Modèle B30) en mode continu pendant deux fois six minutes avec refroidissement. La fraction insoluble est collectée par centrifugation à 12000 g à 4°C pendant 15 minutes puis lavée par du PBS et séchée sous vide à 30°C pendant 15 minutes.

## 3. Dénaturation, réduction et renaturation

Le culot de sonication contenant les produits insolubles provenant de 1 litre de culture est repris dans 4 ml de solution dénaturante et réductrice (6M guanidine-HCl, 0,1M $KH_2PO_4$ pH 7,5, 0,1M β-mercaptoéthanol). La suspension ainsi obtenue est agitée doucement en tube fermé à 4°C pendant 16 heures. Une solution presque limpide est alors obtenue .Un léger précipité insoluble est éliminé par centrifugation. Une dilution au 1/100 du surnageant est effectuée dans une solution de renaturation (50 mM Tris-HCl pH 8,5, 100 mM NaCl, 1 mM EDTA) et ce mélange est laissé à 4°C pendant 24 heures. La solution est ensuite centrifugée pour éliminer une opalescence blanchâtre. Le surnageant obtenu est concentré environ 100 fois par ultrafiltration (membrane à "cut-off" de 30.000 daltons ; par exemple en utilisant les unités d'ultrafiltration à usage unique Millipore CS-30), de nouveau clarifié par centrifugation puis dialysé contre un tampon phosphate (Na) 20 mM pH 7,5. La protéine fusion cII-SAH (pseudo-pro-SAH) ainsi obtenue est homogène à plus de 90 % d'après une analyse par électrophorèse sur gel de polyacrylamide SDS.

## 4. Conversion de la cII-SAH en SAH mature

Une solution de trypsine (préparée par exemple à partir de trypsine lyophilisée pour usage analytique commercialisée par Boehringer Mannheim) est préparée dans la solution de réaction. La cII-SAH est traitée à une concentration, par exemple de l'ordre de 1 mg/ml, avec une quantité de trypsine comprise entre 1/5000 et 1/1000 (rapport massique à la SAH) à 37°C pendant 30 à 60 minutes dans un tampon phosphate (Na) 50 mM pH 7,5, 50 µM $CaCl_2$.

## 5. Vérification de la coupure

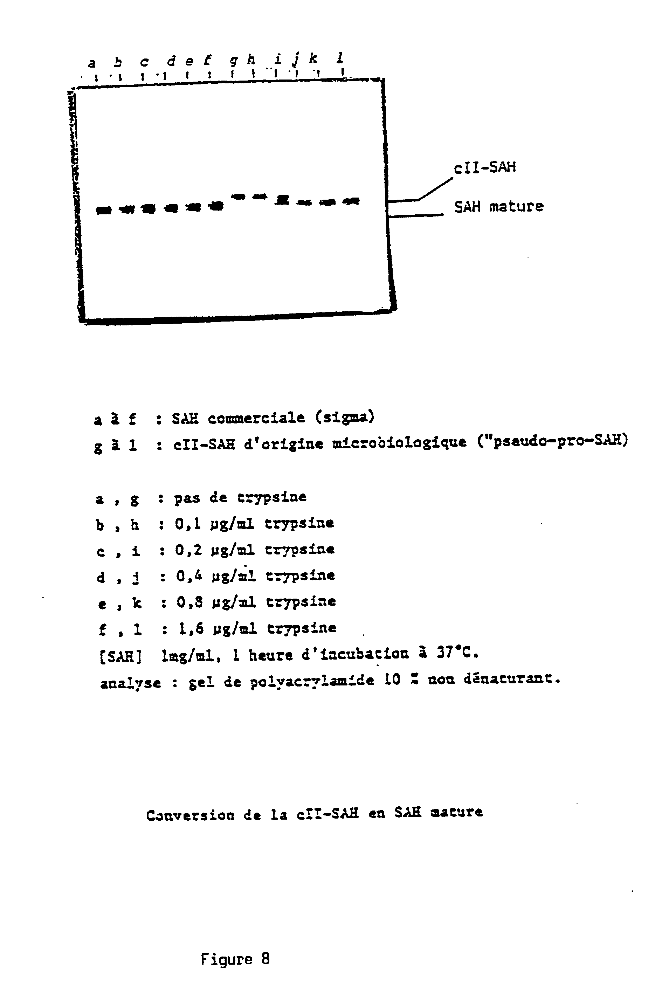

Il est possible de suivre la réaction de conversion par la trypsine sur un gel de polyacrylamide non dénaturant (figure 8). A cause de la présence de plusieurs acides aminés chargés positivement dans l'hexapeptide N-terminal, la migration électrophorétique de la cII-SAH est plus lente sur ce type de gel que celle de la SAH native. Sur la figure 4, on peut voir que la SAH commerciale n'est pas codifiée de façon appréciable par la trypsine dans la gamme de concentrations utilisée. Par contre, la cII-SAH est convertie par l'action de la trypsine en une molécule qui co-migre avec la SAH commerciale. La séquence N-terminale de cette protéine modifiée par la trypsine a été examinée par dégradation de Edman et les résultats obtenus confirment bien que le site de protéolyse est situé après le dipeptide Lys-Arg, à la fin de la partie cII de la protéine hydride. La protéine ainsi générée possède l'acide aspartique comme résidu N-terminal ; elle est donc identique à la SAH d'origine naturelle.

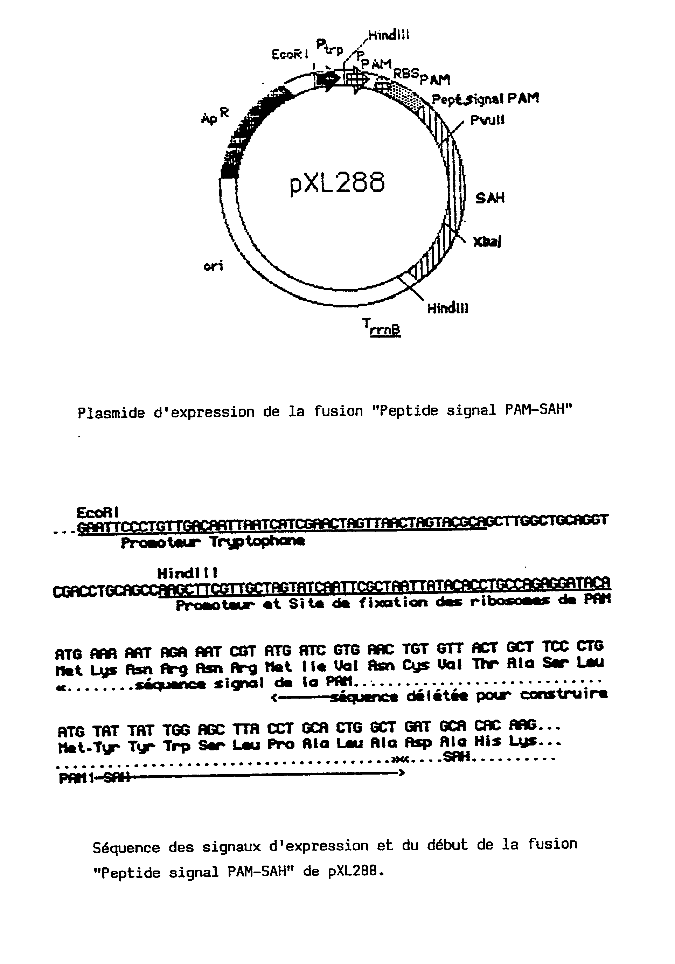

Dans la demande de brevet européen EP 86400618.4, publiée sous le numéro 200590, au nom de la demanderesse, a été décrite la construction du plasmide "pXL288". Après introduction dans une souche appropriée d'E.coli, ce plasmide (figure 9) permet l'expression à haut niveau d'une protéine hybride, non maturée in vivo, constituée par la fusion entre le peptide signal de la pénicilline G amidase (PAM) (EC 3.5.11 ; pénicilline aminohydrolase) de E.coli et la SAH mature.

Le plasmide "pXL288" est caractérisé en ce qu'il contient le promoteur Ptrp de l'opéron tryptophane de E.coli en amont du promoteur de la PAM, le site de fixation des ribosomes du gène de la PAM, le codon d'initiation ATG et les nucléotides du peptide signal de la PAM fusionnés avec le gène de structure de la SAH.

L'extrémité N-terminale du peptide leader de la PAM contient une séquence de 5 acides aminés basiques. Cette basicité constitue une des caractéristiques générales d'un peptide signal de sécrétion (M.E.E. Watson, Nucl. Acids. Res., 12, p, 5145 et suivantes). Il a maintenant été trouvé que les 6 premiers acides aminés de ce peptide signal (Met Lys Asn Arg Asn Arg-, "PAM 1") peuvent jouer le rôle de séquence "pseudo-pro".

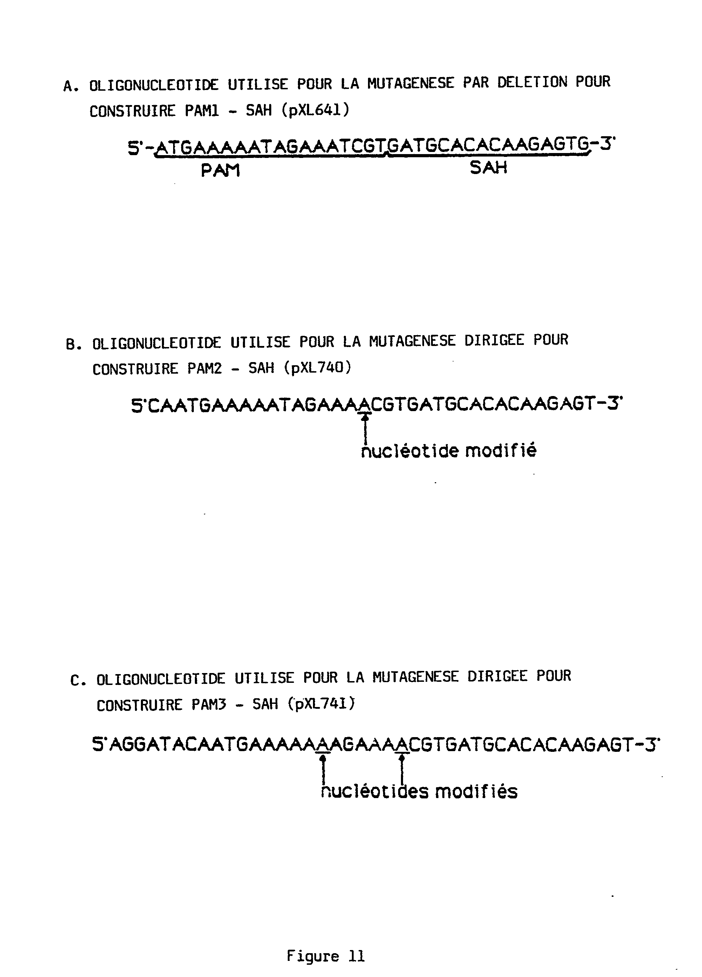

Dans ce but, les nucléotides correspondant aux acides aminés 7 à 26 du peptide leader de la PAM ont été supprimés afin de fusionner exactement la séquence "PAM1" à la séquence de la SAH mature en utilisant la technique de suppression dirigée par oligonucléotide décrite précédemment (figure 9). L'oligonucléotide permettant de réaliser cette suppression est représenté par la figure 11A. La séquence modifiée est ensuite substituée dans le plasmide "pXL288" pour donner le plasmide "pXL641" dont la structure est la suivante : "EcoR1-Ptrp-Sall-[Promoteur PAM-RBS PAM-séquence nucléotidique codant pour PAM1]-gène SAH".

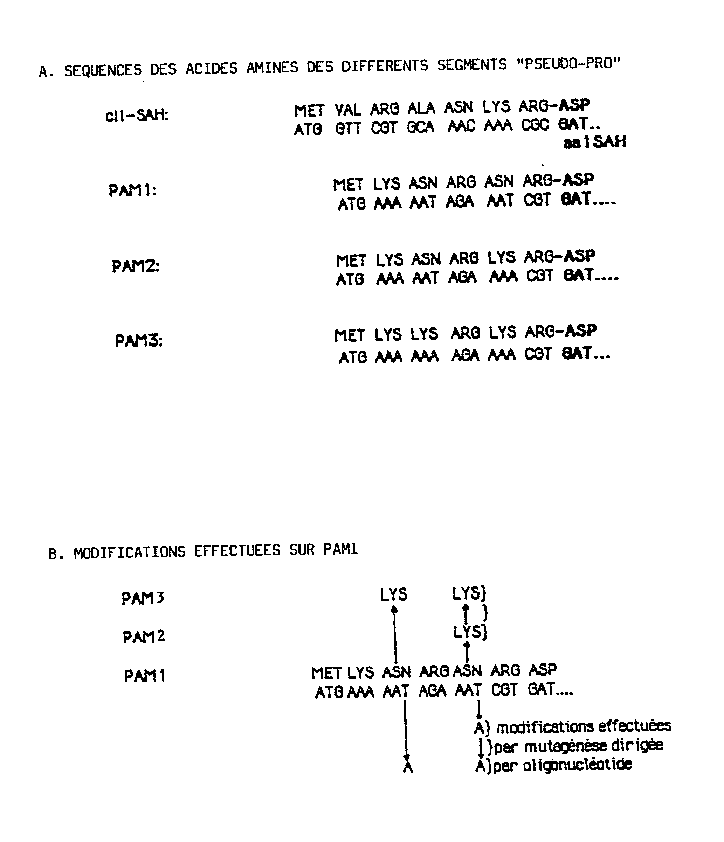

Deux dérivés de la séquence "PAM1" sont construits par mutagénèse dirigée par oligonucléotide, après sous-clonage dans le bactériophage M13mp18amIV, selon la méthode décrite par P. CARTER et coll., Nucl. Acids Res., 1985, 13, p.4431 et suivantes. Les oligonucléotides permettant de réaliser cette mutagénèse sont représentés dans les figures 11B et 11C. Après reconstruction, deux plasmides analogues au plasmide "pXL641" contenant les séquences codant pour "PAM2" (Met Lys Asn Arg Lys Arg- ; plasmide "pXL740") et "PAM3" (Met Lys Lys Arg Lys Arg- ; plasmide "pXL741") sont obtenus (figure 10).

Après introduction des plasmides "pXL641", "pXL740" et "pXL741" dans une souche appropriée de E.coli telle que E.coli 54125 (Collection de l'Institut Pasteur), on obtient des souches produisant respectivement les protéines hybrides PAM1-SAH, PAM2-SAH et PAM3-SAH à des taux de l'ordre de 5 à 10 mg/l de milieu pour une absorbance de 1 à 610 nm en opérant dans les conditions décrites dans la demande de brevet européen EP 86400618.4 (200590).

La protéine hybride se trouve dans la fraction insoluble du lysat cellulaire et peut être renaturée et partiellement purifiée selon les méthodes décrites précédemment. Chaque protéine hybride obtenue après renaturation peut être convertie en SAH mature par digestion ménagée au moyen d'une concentration optimisée de trypsine dans les conditions décrites précédemment.

Conformément aux dispositions du Traité de Budapest, ont été déposés au CBS à Baarn (Pays-Bas) le 3 février 1987 :
- Un échantillon du microorganisme E.coli E103S (pRK 248 cIts) contenant le plasmide pXL 462 (souche G-1398) sous le numéro CBS 143-87.
- Un échantillon du microorganisme E.coli B contenant le plasmide pXL 641 (souche G-2083) sous le numéro CBS 144-87.
- Un échantillon du microorganisme E.coli B contenant le plasmide pXL 740 (souche G-2146) sous le numéro CBS 145-87.
- Un échantillon du microorganisme E.coli B contenant le plasmide pXL 741 (souche G-2147 sous le numéro CBS 146-87.

## Revendications

1. Procédé de préparation de la sérum-albumine humaine mature caractérisé en ce que :
- dans une première étape on prépare une protéine hybride contenant une extension peptidique N-terminale hydrophile de 5 à 8 acides aminés, et de préférence 6 à 7, terminée par un site de coupure par la trypsine, fusionnée avec la séquence peptidique de la sérum-albumine humaine mature, par culture d'une souche d'E.coli capable d'assurer le maintien d'un plasmide contenant la séquence nucléotidique codant pour ladite protéine hybride, dont l'expression est contrôlée par un promoteur bactérien inductible,
- dans une deuxième étape, on convertit la molécule dénaturée et insoluble ainsi obtenue en molécule renaturée et soluble, en utilisant une méthode de dénaturation et renaturation permettant un réarrangement des structures secondaire et tertiaire de la chaîne polypeptidique, et
- dans une troisième étape, on convertit cette protéine hybride par la trypsine en une protéine identique en structure primaire à la sérum-albumine humaine mature.

2. Procédé selon la revendication 1 caractérisé en ce que les codons codant pour l'extension peptidique N-terminale sont choisis parmi les sept premiers codons du gène cII du bactériophage lambda et les six premiers codons du gène de la pénicilline amidase éventuellement transformés par mutagénèse dirigée.

3. Le plasmide "pXL462" déposé sous le numéro CBS 143-87 caractérisé en ce qu'il contient le promoteur P$_L$, le site de fixation des ribosomes du gène cII privé du signal de terminaison de la transcription tR1, le codon d'initiation ATG et les six premiers codons du gène cII fusionnés avec le gène de structure de la sérum-albumine humaine mature.

4. Le plasmide "pXL641" déposé sous le numéro CBS 144-87 caractérisé en ce qu'il contient le promoteur Ptrp suivi du promoteur de la pénicilline amidase, le site de fixation des ribosomes du gène de la pénicilline amidase et les six premiers codons du gène de la pénicilline amidase fusionnés avec le gène de structure de la sérum-albumine humaine mature.

5. Le plasmide "pXL740" déposé sous le numéro CBS 145-87 caractérisé en ce qu'il contient le promoteur Ptrp suivi du promoteur de la pénicilline amidase, le site de fixation des ribosomes du gène de la pénicilline amidase et les six premiers codons d'un gène de la pénicilline amidase modifié par mutagénèse dirigée codant pour le polypeptide Met-Lys-Asn-Arg-Lys-Arg fusionnés avec le gène de structure de la sérum-albumine humaine mature.

6. Le plasmide "pXL741" déposé sous le numéro 146-87 caractérisé en ce qu'il contient le promoteur Ptrp suivi du promoteur de la pénicilline amidase, le site de fixation des ribosomes du gène de la pénicilline amidase et les six premiers codons d'un gène de la pénicilline amidase modifié par mutagénèse dirigée codant pour le polypeptide Met-Lys-Lys-Arg-Lys-Arg fusionnés avec le gène de structure de la sérum-albumine humaine mature.

7. La protéine hybride comprenant une extension peptidique N-terminale hydrophile de 5 à 8 acides aminés, et de préférence 6 à 7, terminée par un site de coupure par la trypsine, fusionnée avec la séquence peptidique de la sérum-albumine humaine mature lorsqu'elle est obtenue par culture d'une souche d'E. Coli selon la revendication 1.

8. La protéine hybride selon la revendication 7 comprenant à l'extrémité N-terminale les sept premiers acides aminés de la protéine cII de bactériophage lambda, (Met)-Val-Arg-Ala-Asn-Lys-Arg, fusionnés avec la séquence peptidique de la sérum-albumine humaine mature lorsqu'elle est obtenue par culture d'une souche d'E.Coli capable d'assurer le maintien du plasmide "pXL462" défini dans la revendication 3.

9. La protéine hybride selon la revendication 7 comprenant à l'extrémité N-terminale les six premiers acides aminés de la pénicilline amidase, Met-Lys-Asn-Arg-Asn-Arg, fusionnés avec la séquence peptidique de la sérum-albumine humaine mature, lorsqu'elle est obtenue par culture d'une souche d'E.Coli capable d'assurer le maintien du plasmide "pXL641" défini dans la revendication 4.

10. La protéine hybride selon la revendication 7 comprenant à l'extrémité N-terminale les six premiers acides aminés d'une pénicilline amidase N-terminale modifiée par mutagénèse dirigée, Met-Lys-Asn-Arg-Lys-Arg, fusionnés à la séquence peptidique de la sérum-albumine humaine mature, lorsqu'elle est obtenue par culture d'une souche d'E.coli capable d'assurer le maintien du plasmide "pXL740" défini dans la revendication 5.

11. La protéine hybride selon la revendication 7 comprenant à l'extrémité N-terminale les six premiers acides aminés d'une pénicilline amidase modifiée par mutagénèse dirigée, Met-Lys-Lys-Arg-Lys-Arg, fusionnés avec la séquence peptidique de la sérum-albumine humaine mature, lorsqu'elle est obtenue par culture d'une souche d'E.coli capable d'assurer le maintien du plasmide "pXL741" défini dans la revendication 6.

## Claims

1. Process for the preparation of mature human serum albumin, characterised in that:
– in a first stage a hybrid protein is prepared containing a hydrophilic N-terminal peptide extension containing 5 to 8, and preferably 6 to 7, amino acids, terminated by a site for cutting with trypsin, which is fused with the peptide sequence of mature human serum albumin, by culture of a strain of E.coli capable of ensuring the conservation of a plasmid containing the nucleotide sequence coding for the said hybrid protein, whose expression is controlled by an inducible bacterial promoter,
– in a second stage the denatured and insoluble molecule thus obtained is converted into a renatured and soluble molecule by using a denaturing and renaturing method permitting a rearrangement of the secondary and tertiary structures of the polypeptide chain, and
– in a third stage this hybrid protein is converted by trypsin into a protein identical in primary structure to mature human serum albumin.

2. Process according to Claim 1, characterised in that the codons coding for the N-terminal peptide extension are chosen from the first seven codons of the lambda bacteriophage cII gene and the first six codons of the penicillin amidase gene, optionally transformed by directed mutagenesis.

3. The plasmid "pXL462" deposited under number CBS 143-87, characterised in that it contains the $P_L$ promoter, the ribosome binding site of the cII gene deprived of the tR1 transcription termination signal, the ATG initiation codon and the first six codons of the cII gene fused with the structural gene of mature human serum albumin.

4. The plasmid "pXL641" deposited under number CBS 144-87, characterised in that it contains the Ptrp promoter followed by the penicillin amidase promoter, the ribosome binding site of the penicillin amidase gene and the first six codons of the penicillin amidase gene which are fused with the structural gene of mature human serum albumin.

5. The plasmid "pXL740" deposited under number CBS 145-87, characterised in that it contains the Ptrp promoter followed by the penicillin amidase promoter, the ribosome binding site of the penicillin amidase gene and the first six codons of a penicillin amidase gene modified by directed mutagenesis coding for the Met-Lys-Asn-Arg-Lys-Arg polypeptide fused with the structural gene of mature human serum albumin.

6. The plasmid "pXL741" deposited under number 146-87, characterised in that it contains the Ptrp promoter followed by the penicillin amidase promoter, the ribosome binding site of the penicillin amidase gene and the first six codons of a penicillin amidase gene modified by directed mutagenesis coding for the Met-Lys-Lys-Arg-Lys-Arg polypeptide fused with the structural gene of mature human serum albumin.

7. The hybrid protein comprising a hydrophilic N-terminal peptide extension containing 5 to 8, and preferably 6 to 7, amino acids, terminated by a site for cutting with trypsin, which is fused with the peptide sequence of mature human serum albumin, when it is produced by culture of a strain of E.coli according to Claim 1.

8. The hybrid protein according to Claim 7, comprising at the N-terminal end the first seven amino acids of the lambda bacteriophage cII protein, (Met)-Val-Arg-Ala-Asn-Lys-Arg, which are fused with the peptide sequence of mature human serum albumin, when it is produced by culture of a strain of E.coli capable of ensuring the conservation of the plasmid "pXL462" defined in Claim 3.

9. The hybrid protein according to Claim 7, comprising at the N-terminal end the first six amino acids of penicillin amidase, Met-Lys-Asn-Arg-Asn-Arg, fused with the peptide sequence of mature human serum albumin, when it is produced by culture of a strain of E.coli capable of ensuring the conservation of the plasmid "pXL641" defined in Claim 4.

10. The hybrid protein according to Claim 7, comprising at the N-terminal end the first six N-terminal amino acids of a penicillin amidase modified by directed mutagenesis, Met-Lys-Asn-Arg-Lys-Arg, fused to the peptide sequence of mature human serum albumin, when it is produced by culture of a strain of E.coli capable of ensuring the conservation of the plasmid "pXL740" defined in Claim 5.

11. The hybrid protein according to Claim 7, comprising at the N-terminal end the first six amino acids of a penicillin amidase modified by directed mutagenesis, Met-Lys-Lys-Arg-Lys-Arg, fused with the peptide sequence of mature human serum albumin, when it is produced by culture of a strain of E.coli capable of ensuring the conservation of the plasmid "pXL741" defined in Claim 6.


**Patentansprüche**

1. Verfahren zur Herstellung von reifem menschlichem Serum-albumin, dadurch gekennzeichnet, daß man
– in einer ersten Stufe ein Hybridprotein herstellt, das eine hydrophile N-endständige Peptidverlängerung von 5 bis 8, vorzugsweise 6 bis 7 Aminosäuren, abgeschlossen durch eine Trypsinschnittstelle, fusioniert mit der Peptidsequenz des reifen menschlichen Serumalbumins, herstellt durch Züchtung eines Stammes von E.coli, der den Bestand eines Plasmids zu gewährleisten vermag, das die für das Hybridprotein, dessen Expression durch einen induzierbaren bakteriellen Promotor regelbar ist, codierende Nukleotidsequenz enthält,
– in einer zweiten Stufe das so erhaltene denaturierte und unlösliche Molekül in ein renaturiertes und lösliches Molekül umwandelt, indem man eine Denaturierungs- und Renaturierungs-methode anwendet, die eine Umlagerung sekundärer und tertiärer Strukturen der Polypeptidkette ermöglicht, und
– in einer dritten Stufe dieses Hybridprotein mit Trypsin in ein Protein umwandelt, das in der Primärstruktur dem reifen menschlichen Serumalbumin identisch ist.

2. Verfahren nach Anspruch 1, dadurch gekennzeichnet, daß die die N-endständige Peptidverlängerung codierenden Codons aus gewählt sind aus den sieben ersten Codons des Gens cII des Bakteriophagen Lambda und den sechs ersten Codons des Gens von Penicillinamidase, gegebenenfalls transformiert durch gerichtete Mutagenese.

3. Plasmid "pXL462", hinterlegt unter der Nummer CBS 143-87, dadurch gekennzeichnet, daß es den Promotor $P_L$, die Bindestelle der Ribosomen des Gens cII, abgeschlossen durch das Transkriptionsterminationssignal tR1, das Startcodon ATG und die sechs ersten Codons des Gens cII, fusioniert mit dem Strukturgen des reifen menschlichen Serumalbumins, enthält.

4. Plasmid "pXL641", hinterlegt unter der Nummer CBS 144-87, dadurch gekennzeichnet, daß es den Promotor Ptrp, gefolgt vom Promotor der Penicillinamidase, die Bindestelle der Ribosomen des Gens der Penicillinamidase und die sechs ersten Codons des Gens der Penicillinamidase, fusioniert mit dem Strukturgen des reifen menschlichen Serumalbumins, enthält.

5. Plasmid "pXL740", hinterlegt unter der Nummer CBS 145-87, dadurch gekennzeichnet, daß es den Promotor Ptrp, gefolgt vom Promotor der Penicillinamidase, die Bindestelle der Ribosomen des Gens der Penicillinamidase und die sechs ersten Codons des Gens der durch gerichtete Mutagenese modifizierten Penicillinamidase, die für das Polypeptid Met-Lys-Asn-Arg-Lys-Arg codieren, fusioniert mit dem Strukturgen des reifen menschlichen Serumalbumins, enthält.

6. Plasmid "pXL741", hinterlegt unter der Nummer CBS 146-87, dadurch gekennzeichnet, daß es den Promotor Ptrp, gefolgt vom Promotro der Penicillinamidase, die Bindestelle der Ribosomen des Gens der Penicillinamidase und die sechs ersten Codons eines Gens der durch gerichtete Mutagenese modifizierten Penicillinamidase, die für das Polypeptid Met-Lys-Lys-Arg-Lys-Arg codieren, fusioniert mit dem Strukturgen des reifen menschlichen Serumalbumins, enthält.

7. Hybridprotein, umfassend eine hydrophile N-endständige Peptidverlängerung von 5 bis 8, vorzugsweise 6 bis 7 Aminosäuren, abgeschlossen durch eine Trypsinschnittstelle, fusioniert mit der Peptidsequenz reifen menschlichen Serum-albumins, erhalten durch Züchtung eines Stammes von E.coli nach Anspruch 1.

8. Hybridprotein nach Anspruch 7, umfassend am N-endständigen Ende die sieben ersten Aminosäuren des Proteins cll des Bakteriophagen Lambda, (Met)-Val-Arg-Ala-Asn-Lys-Arg, fusioniert mit der Peptidsequenz des reifen menschlichen Serumalbumins, erhalten durch Züchtung eines Stammes von E.coli, der den Bestand des im Anspruch 3 definierten Plasmids "pXL462" zu gewährleisten vermag.

9. Hybridprotein nach Anspruch 7, umfassend am N-endständigen Ende die sechs ersten Aminosäuren der Penicillinamidase, Met-Lys-Asn-Arg-Asn-Arg, fusioniert mit der Peptidsequenz des reifen menschlichen Serumalbumins, erhalten durch Züchtung eines Stammes von E.coli, der den Bestand des im Anspruch 4 definierten Plasmids "pXL641" zu gewährleisten vermag.

10. Hybridprotein nach Anspruch 7, umfassend am N-endständigen Ende die sechs ersten Aminosäuren der durch gerichtete Mutagenese modifizierten Penicillinamidase, Met-Lys-Asn-Arg-Lys-Arg, fusioniert mit der Peptidsequenz des reifen menschlichen Serumalbumins, erhalten durch Züchtung eines Stammes von E.coli, der den Bestand des im Anspruch 5 definierten Plasmids "pXL740" zu gewährleisten vermag.

11. Hybridprotein nach Anspruch 7, umfassend am N-enständigen Ende die sechs ersten Aminosäuren der durch gerichtete Mutagenese modifizierten Penicillinamidase, Met-Lys-Lys-Arg-Lys-Arg, fusioniert mit der Peptidsequenz des reifen menschlichen Serumalbumins, erhalten duch Züchtung eines Stammes von E.coli, der den Bestand des im Anspruch 6 definierten Plasmids "pXL741" zu gewährleisten vermag.

Met lys trp val thr phe ile ser leu leu phe leu phe ser ser ala tyr ser arg gly val phe arg arg asp ala ...

1

STUCTURE DE LA "PREPRO-SAH"

FIGURE 1

Carte de restriction du gène de l'albumine humaine et position des insertions

Le chiffre 1 correspond au 1er acide aminé de l'albumine humaine.
L'insertion du plasmide "pTlBll" s'étend au-delà de l'extrémité 5',
vers la séquence de la proalbumine.

EP 0 236 210 B1

Figure 3

Figure 3

Figure 3

FIGURE 3

SEQUENCE DE L'INSERTION DE pXL53

                10        20        30        40        50        60        70        80

EcoRI
GAATTCCTCACTCATTAGGCACCCCCAGGCTTTTACACATTTATGCTTCCGGCTCGTATGTTGTGTGGAATTGTGAGCGG

CTTAAGGAGTGAGTAATCCGTGGGGGTCCGAAAATGTGTAAATACGAAGGCCGAGCATACAACACACCTTAACACTCGCC


                90        100       110         ↓ 120      130       140       150       160

ATAACAATTTCACACAGGAAACAGGAATCCATGGATGCACACAAGAGTGAGGTTGCTCATCGGTTTAAAGATTTGCGAGA

TATTGTTAAAGTGTGTCCTTTGTCCTTAGGTACCTACGTGTGTTCTCACTCCAACGAGTAGCCAAATTTCTAAACCCTCT


                170       180       190       200       210       220       230       240

AGAAAATTTCAAAGCCTTGGTGTTGATTGCCTTTGCTCAGTATCTTCAGCAGTGTCCATTTGAAGATCATGTAAAATTAG

TCTTTTAAAGTTTCGGAACCACAACTAACGGAAACGAGTCATAGAAGTCGTCACAGGTAAACTTCTAGTACATTTTAATC

Figure 4

EP 0 236 210 B1

```
        250       260       270       280       290       300       310       320
TGAATGAAGTAACTGAATTTGCAAAAACATGTGTTGCTGATGAGTCAGCTGAAAATTGTGACAAATCACTTCATACCCTT
ACTTACTTCATTGACTTAAACGTTTTTGTACACAACGACTACTCAGTCGACTTTTAACACTGTTAGTGAAGTATGGGAA


        330       340       350       360       370       380       390       400
TTTGGAGACAAATTATGCACAGTTGCAACTCTTCGTGAAACCTATGGTGAAATGGCTGACTGCTGTGCAAAACAAGAACC
AAACCTCTGTTTAATACGTGTCAACGTTGAGAAGCACTTTGGATACCACTTTACCGACTGACGACACGTTTTGTTCTTGG


        410       420       430       440       450       460       470       480
TGAGAGAAATGAATGCTTCTTGCAACACAAAGATGACAATCCAAATCTCCCCCGATTGGTGAGACCAGAGGTTGATGTGA
ACTCTCTTTACTTACGAAGAACGTTGTGTTTCTACTGTTAGGTTTAGAGGGGGCTAACCACTCTGGTCTCCAACTACACT


        490       500       510       520       530       540       550       560
TGTGCACTGCTTTTCATGACAATGAAGAGACATTTTTGAAAAAATACTTATATGAAATTGCCAGAAGACATCCTTACTTT
ACACGTGACGAAAAGTACTGTTACTTCTCTGTAAAAACTTTTTTATGAATATACTTTAACCGTCTTCTGTAGGAATGAAA
```

Figure 4 (suite)

21

TATGCCCCGGAACTCCTTTTCTTTGCTAAAAGGTATAAAGCTGCTTTTACAGAATGTTGCCAAGCTGCTGATAAAGCAGC

ATACGGGGCCTTGACGAAAAGAAACGATTTTCCATATTTCGACGAAAATGTCTTACAACGGTTCGACGACTATTTCGTCG·

CTGCCTGTTGCCAAAGCTCGATGAACTTCGGGATGAAGGGAAGGCTTCGTCTGCCAAACAGAGACTCAAGTGTGCCAGTC

GACCGACAACGGTTTCGAGCTACTTGAAGCCCTACTTCCCTTCCGAAGCAGACGGTTTGTCTCTGAGTTCACACGGTCAG

TCCAAAAATTTGGAGAAAGAGCTTTCAAAGCATGGGCAGTAGCTCGCCTGAGCCAGAGATTTCCCAAAGCTGAGTTTGCA

AGGTTTTTAAACCTCTTTCTCGAAAGTTTCGTACCCGTCATCGAGCGGACTCGGTCTCTAAAGGGTTTCGACTCAAACGT

GAAGTTTCCAAGTTAGTGACAGATCTTACCAAAGTCCACACGGAATGCTGCCATGGAGATCTGCTTGAATGTGCTGATGA

CTTCAAAGGTTCAATCACTGTCTAGAATGGTTTCAGGTGTGCCTTACGACGGTACCTCTAGACGAACTTACACGACTACT

EP 0 236 210 B1

Figure 4 (suite)

EP 0 236 210 B1

870     900     910     920     930     940     950     960

CAGGGCGGACCTTGCCAAGTATATCTGTGAAAATCAAGATTCGATCTCCAGTAAACTGAAGGAATGCTGTGAAAAACCTC

GTCCCGCCTGGAACGGTTCATATAGACACTTTTAGTTCTAAGCTAGAGGTCATTTGACTTCCTTACGACACTTTTTGGAG

970     980     990     1000     1010     1020     1030     1040

TGTTGGAAAAATCCCACTGCATTGCCGAAGTGGAAAATGATGAGATGCCTGCTGACTTGCCTTCATTAGCGGCTGATTTT

ACAACCTTTTTAGGGTGACGTAACGGCTTCACCTTTTACTACTCTACGGACGACTGAACGGAAGTAATCGCCGACTAAAA

1050     1060     1070     1080     1090     1100     1110     1120

GTTGAAAGTAAGGATGTTTGCAAAAACTATGCTGAGGCAAAGGATGTCTTCTTGGGCATGTTTTTGTATGAATATGCAAG

CAACTTTCATTCCTACAAACGTTTTTGATACGACTCCGTTTCCTACAGAAGAACCCGTACAAAAACATACTTATACGTTC

1130     1140     1150     1160     1170     1180     1190     1200

AAGGCATCCTGATTACTCTGTCGTACTGCTGCTGAGACTTGCCAAGACATATGAAACCACTCTAGAGAAGTGCTGTGCCG

TTCCGTAGGACTAATGAGACAGCATGACGACGACTCTGAACGGTTCTGTATACTTTGGTGAGATCTCTTCACGACACGGC

Figure 4 (suite)

EP 0 236 210 B1

1210    1220    1230    1240    1250    1260    1270    1280

CTGCAGATCCTCATGAATGCTATGCCAAAGTGTTCGATGAATTTAAACCTCTTATGGAAGAGCCTCAGAATTTAATCAAA

GACGTCTAGGAGTACTTACGATACGGTTTCACAAGCTACTTAAATTTGGAGAATACCTTCTCGGAGTCTTAAATTAGTTT


1290    1300    1310    1320    1330    1340    1350    1360

CAAAATTGTGAGCTTTTTGAGCAGCTTGGAGAGTACAAATTCCAGAATGCGCTATTAGTTCGTTACACCAAGAAAGTACC

GTTTTAACACTCGAAAAACTCGTCGAACCTCTCATGTTTAAGGTCTTACGCGATAATCAAGCAATGTGGTTCTTTCATGG


1370    1380    1390    1400    1410    1420    1430    1440

CCAAGTGTCAACTCCAACTCTTGTAGAGGTCTCAAGAAACCTAGGAAAAGTGGGCAGCAAATGTTGTAAACATCCTGAAG

GGTTCACAGTTGAGGTTGAGAACATCTCCAGAGTTCTTTGGATCCTTTTCACCCGTCGTTTACAACATTTGTAGGACTTC


1450    1460    1470    1480    1490    1500    1510    1520

CAAAAAGAATGCCCTGTGCAGAAGACTATCTATCCGTGGTCCTGAACCAGTTATGTGTGTTGCATGAGAAAACGCCAGTA

GTTTTTCTTACGGGACACGTCTTCTGATAGATAGGCACCAGGACTTGGTCAATACACACAACGTACTCTTTTGCGGTCAT

Figure 4 (suite)

EP 0 236 210 B1

Figure 4 (suite)

1530      1540      1550      1560      1570      1580      1590      1600

AGTGACAGAGTCACCAAATGCTGCACAGAATCCTTGGTGAACAGGCGACCATGCTTTTCAGCTCTGGAAGTCGATGAAAC

TCACTGTCTCAGTGGTTTACGACGTGTCTTAGGAACCACTTGTCCGCTGGTACGAAAAGTCGAGACCTTCAGCTACTTTG

1610      1620      1630      1640      1650      1660      1670      1680

ATACGTTCCCAAAGAGTTTAATGCTGAAACATTCACCTTCCATGCAGATATATGCACACTTTCTGAGAAGGAGAGACAAA

TATGCAAGGGTTTCTCAAATTACGACTTTGTAAGTGGAAGGTACGTCTATATACGTGTGAAAGACTCTTCCTCTCTGTTT

1690      1700      1710      1720      1730      1740      1750      1760

TCAAGAAACAAACTGCACTTGTTGAGCTTGTGAAACACAAGCCCAAGGCAACAAAAGAGCAACTGAAAGCTGTTATGGAT

AGTTCTTTGTTTGACGTGAACAACTCGAACACTTTGTGTTCGGGTTCCGTTGTTTTCTCGTTGACTTTCGACAATACCTA

1770      1780      1790      1800      1810      1820      1830      1840

GATTTCGCAGCTTTTGTAGAGAAGTGCTGCAAGGCTGACGATAAGGAAACCTGCTTTGCCGAGGAGGGTAAAAAACTTGT

CTAAAGCGTCGAAAACATCTCTTCACGACGTTCCGACTGCTATTCCTTTGGACGAAACGGCTCCTCCCATTTTTTGAACA

EP 0 236 210 B1

Figure 4 (suite)

585

```
      1850         1860         1870         1880         1890         1900         1910         1920
TGCTGCAAGTCAAGCTGCCTTAGGCTTATAACATCACATTTAAAAGCATCTCAGCCTACCATGAGAATAAGAGAAAGAAA
ACGACGTTCAGTTCGACGGAATCCGAATATTGTAGTGTAAATTTTCGTAGAGTCGGATGGTACTCTTATTCTCTTTCTTT


      1930         1940         1950         1960         1970         1980         1990         2000
ATGAAGATCAAAAGCTTATTCATTCTGTTTTTCTTTTTCGTTGGTGTAAAAGCCAACACCCTGTCTAAAAAACATAAATT
TACTTCTAGTTTTCGAATAAGTAAGACAAAAAGAAAAAGCAACCACATTTTCGGTTGTGGGACAGATTTTTTGTATTTAA


      2010         2020         2030         2040         2050         2060         2070         2080
TCTTTAATCATTTTAATCATTTTGCCTCTTTTCTCTGTGCTTCAATTAATAAAAAATGGAAAGAATCTAAAAAAACCCCC
AGAAATTAGTAAAATTAGTAAAACGGAGAAAAGAGACACGAAGTTAATTATTTTTTACCTTTCTTAGATTTTTTTGGGGG


      2090         2100         2110         2120         2130         2140         2150         2160
            PstI
CCCCCCCCCCCCCTGCAGCAATAGCAACAACGTTGCGCAAACTATTAACTGGCGAA
GGGGGGGGGGGGGACGTCGTTATCGTTGTTGCAACGCGTTTGATAATTGACCGCTT
```

TRADUCTION DU GENE DE L'ALBUMINE HUMAINE DANS pXL53

                    125                     140                     155                     170
ATG GAT GCA CAC AAG AGT GAG GTT GCT CAT CGG TTT AAA GAT TTG GGA GAA GAA AAT TTC
MET ASP ALA HIS LYS SER GLU VAL ALA HIS ARG PHE LYS ASP LEU GLY GLU GLU ASN PHE
①

                    185                     200                     215                     230
AAA GCC TTG GTG TTG ATT GCC TTT GCT CAG TAT CTT CAG CAG TGT CCA TTT GAA GAT CAT
LYS ALA LEU VAL LEU ILE ALA PHE ALA GLN TYR LEU GLN GLN CYS PRO PHE GLU ASP HIS

                    245                     260                     275                     290
GTA AAA TTA GTG AAT GAA GTA ACT GAA TTT GCA AAA ACA TGT GTT GCT GAT GAG TCA GCT
VAL LYS LEU VAL ASN GLU VAL THR GLU PHE ALA LYS THR CYS VAL ALA ASP GLU SER ALA

                    305                     320                     335                     350
GAA AAT TGT GAC AAA TCA CTT CAT ACC CTT TTT GGA GAC AAA TTA TGC ACA GTT GCA ACT
GLU ASN CYS ASP LYS SER LEU HIS THR LEU PHE GLY ASP LYS LEU CYS THR VAL ALA THR

EP 0 236 210 B1

Figure 5

EP 0 236 210 B1

Figure 5 (suite)

365   380   395   410

CTT CGT GAA ACC TAT GGT GAA ATG GCT GAC TGC TGT GCA AAA CAA GAA CCT GAG AGA AAT

LEU ARG GLU THR TYR GLY GLU MET ALA ASP CYS CYS ALA LYS GLN GLU PRO GLU ARG ASN

425   440   455   470

GAA TGC TTC TTG CAA CAC AAA GAT GAC AAT CCA AAT CTC CCC CGA TTG GTG AGA CCA GAG

GLU CYS PHE LEU GLN HIS LYS ASP ASP ASN PRO ASN LEU PRO ARG LEU VAL ARG PRO GLU

485   500   515   530

GTT GAT GTG ATG TGC ACT GCT TTT CAT GAC AAT GAA GAG ACA TTT TTG AAA AAA TAC TTA

VAL ASP VAL MET CYS THR ALA PHE HIS ASP ASN GLU GLU THR PHE LEU LYS LYS TYR LEU

545   560   575   590

TAT GAA ATT GCC AGA AGA CAT CCT TAC TTT TAT GCC CCG GAA CTC CTT TTC TTT GCT AAA

TYR GLU ILE ALA ARG ARG HIS PRO TYR PHE TYR ALA PRO GLU LEU LEU PHE PHE ALA LYS

Figure 5 (suite)

605                    620                  635                  650

AGG TAT AAA GCT GCT TTT ACA GAA TGT TGC CAA GCT GCT GAT AAA GCA GCC TGC CTG TTG

ARG TYR LYS ALA ALA PHE THR GLU CYS CYS GLN ALA ALA ASP LYS ALA ALA CYS LEU LEU


665                    680                  695                  710

CCA AAG CTC GAT GAA CTT CGG GAT GAA GGG AAG GCT TCG TCT GCC AAA CAG AGA CTC AAG

PRO LYS LEU ASP GLU LEU ARG ASP GLU GLY LYS ALA SER SER ALA LYS GLN ARG LEU LYS


725                    740                  755                  770

TGT GCC AGT CTC CAA AAA TTT GGA GAA AGA GCT TTC AAA GCA TGG GCA GTA GCT CGC CTG

CYS ALA SER LEU GLN LYS PHE GLY GLU ARG ALA PHE LYS ALA TRP ALA VAL ALA ARG LEU


785                    800                  815                  830

AGC CAG AGA TTT CCC AAA GCT GAG TTT GCA GAA GTT TCC AAG TTA GTG ACA GAT CTT ACC

SER GLN ARG PHE PRO LYS ALA GLU PHE ALA GLU VAL SER LYS LEU VAL THR ASP LEU THR

EP 0 236 210 B1

Figure 5 (suite)

845                          860                          875                          890

AAA GTC CAC ACG GAA TGC TGC CAT GGA GAT CTG CTT GAA TGT GCT GAT GAC AGG GCG GAC

LYS VAL HIS THR GLU CYS CYS HIS GLY ASP LEU LEU GLU CYS ALA ASP ASP ARG ALA ASP

905                          920                          935                          950

CTT GCC AAG TAT ATC TGT GAA AAT CAA GAT TCG ATC TCC AGT AAA CTG AAG GAA TGC TGT

LEU ALA LYS TYR ILE CYS GLU ASN GLN ASP SER ILE SER SER LYS LEU LYS GLU CYS CYS

965                          980                          995                          1010

GAA AAA CCT CTG TTG GAA AAA TCC CAC TGC ATT GCC GAA GTG GAA AAT GAT GAG ATG CCT

GLU LYS PRO LEU LEU GLU LYS SER HIS CYS ILE ALA GLU VAL GLU ASN ASP GLU MET PRO

1025                         1040                         1055                         1070

GCT GAC TTG CCT TCA TTA GCG GCT GAT TTT GTT GAA AGT AAG GAT GTT TGC AAA AAC TAT

ALA ASP LEU PRO SER LEU ALA ALA ASP PHE VAL GLU SER LYS ASP VAL CYS LYS ASN TYR

EP 0 236 210 B1

1085    1100    1115    1130

GCT GAG GCA AAG GAT GTC TTC TTG GGC ATG TTT TTG TAT GAA TAT GCA AGA AGG CAT CCT
ALA GLU ALA LYS ASP VAL PHE LEU GLY MET PHE LEU TYR GLU TYR ALA ARG ARG HIS PRO

1145    1160    1175    1190

GAT TAC TCT GTC GTA CTG CTG CTG AGA CTT GCC AAG ACA TAT GAA ACC ACT CTA GAG AAG
ASP TYR SER VAL VAL LEU LEU LEU ARG LEU ALA LYS THR TYR GLU THR THR LEU GLU LYS

1205    1220    1235    1250

TGC TGT GCC GCT GCA GAT CCT CAT GAA TGC TAT GCC AAA GTG TTC GAT GAA TTT AAA CCT
CYS CYS ALA ALA ALA ASP PRO HIS GLU CYS TYR ALA LYS VAL PHE ASP GLU PHE LYS PRO

1265    1280    1295    1310

CTT ATG GAA GAG CCT CAG AAT TTA ATC AAA CAA AAT TGT GAG CTT TTT GAG CAG CTT GGA
LEU MET GLU GLU PRO GLN ASN LEU ILE LYS GLN ASN CYS GLU LEU PHE GLU GLN LEU GLY

Figure 5 (suite)

EP 0 236 210 B1

Figure 5 (suite)

1325    1340    1355    1370

GAG TAC AAA TTC CAG AAT GCG CTA TTA GTT CGT TAC ACC AAG AAA GTA CCC CAA GTG TCA

GLU TYR LYS PHE GLN ASN ALA LEU LEU VAL ARG TYR THR LYS LYS VAL PRO GLN VAL SER

1385    1400    1415    1430

ACT CCA ACT CTT GTA GAG GTC TCA AGA AAC CTA GGA AAA GTG GGC AGC AAA TGT TGT AAA

THR PRO THR LEU VAL GLU VAL SER ARG ASN LEU GLY LYS VAL GLY SER LYS CYS CYS LYS

1445    1460    1475    1490

CAT CCT GAA GCA AAA AGA ATG CCC TGT GCA GAA GAC TAT CTA TCC GTG GTC CTG AAC CAG

HIS PRO GLU ALA LYS ARG MET PRO CYS ALA GLU ASP TYR LEU SER VAL VAL LEU ASN GLN

1505    1520    1535    1550

TTA TGT GTG TTG CAT GAG AAA ACG CCA GTA AGT GAC AGA GTC ACC AAA TGC TGC ACA GAA

LEU CYS VAL LEU HIS GLU LYS THR PRO VAL SER ASP ARG VAL THR LYS CYS CYS THR GLU

Figure 5 (suite)

```
        1565                    1580                    1595                    1610
TCC TTG GTG AAC AGG CGA CCA TGC TTT TCA GCT CTG GAA GTC GAT GAA ACA TAC GTT CCC
SER LEU VAL ASN ARG ARG PRO CYS PHE SER ALA LEU GLU VAL ASP GLU THR TYR VAL PRO


        1625                    1640                    1655                    1670
AAA GAG TTT AAT GCT GAA ACA TTC ACC TTC CAT GCA GAT ATA TGC ACA CTT TCT GAG AAG
LYS GLU PHE ASN ALA GLU THR PHE THR PHE HIS ALA ASP ILE CYS THR LEU SER GLU LYS


        1685                    1700                    1715                    1730
GAG AGA CAA ATC AAG AAA CAA ACT GCA CTT GTT GAG CTT GTG AAA CAC AAG CCC AAG GCA
GLU ARG GLN ILE LYS LYS GLN THR ALA LEU VAL GLU LEU VAL LYS HIS LYS PRO LYS ALA


        1745                    1760                    1775                    1790
ACA AAA GAG CAA CTG AAA GCT GTT ATG GAT GAT TTC GCA GCT TTT GTA GAG AAG TGC TGC
THR LYS GLU GLN LEU LYS ALA VAL MET ASP ASP PHE ALA ALA PHE VAL GLU LYS CYS CYS
```

1805 1820 1835 1850

AAG GCT GAC GAT AAG GAA ACC TGC TTT GCC GAG GAG GGT AAA AAA CTT GTT GCT GCA AGT
LYS ALA ASP ASP LYS GLU THR CYS PHE ALA GLU GLU GLY LYS LYS LEU VAL ALA ALA SER

1865 1880 1895

CAA GCT GCC TTA GGC TTA TAA CAT CAC ATT TAA AAG CAT CTC AGC CTA CCA
GLN ALA ALA LEU GLY LEU

585 - STOP

Figure 5 (suite)

34

A. Oligonucléotide codant pour les 6 premiers codons du gène cII

MetVal Arg Ala Asn Lys Arg
5'-AGCTTCATATGGTTCGTGCAAACAAACGCG-3'
3'-AGTATACCAAGCACGTTTGTTTGCGCAGCT.-5'

B. Oligonucléotide utilisé pour la Mutagénèse par délétion.

5'-TCGTGCAAACAAACGCGCATGCACACAAGAGT-3'
        cII                    SAH

OLIGONUCLEOTIDES SYNTHETIQUES
EMPLOYES DANS LA CONSTRUCTION DE
LA cII-SAH

Figure 6

PLASMIDE D'EXPRESSION "PSEUDO-PRO-SAH"

Figure 7

a à f   : SAH commerciale (sigma)

g à l   : cII-SAH d'origine microbiologique ("pseudo-pro-SAH)


a , g   : pas de trypsine

b , h   : 0,1 µg/ml trypsine

c , i   : 0,2 µg/ml trypsine

d , j   : 0,4 µg/ml trypsine

e , k   : 0,8 µg/ml trypsine

f , l   : 1,6 µg/ml trypsine

[SAH]  1mg/ml, 1 heure d'incubation à 37°C.

analyse : gel de polyacrylamide 10 % non dénaturant.



Conversion de la cII-SAH en SAH mature



Figure 8

Plasmide d'expression de la fusion "Peptide signal PAM-SAH"

EcoRI
...GAATTCCCTGTTGACAATTAATCATCGAACTAGTTAACTAGTACGCAGCTTGGCTGCAGGT
        Promoteur Tryptophane

                HindIII
CGACCTGCAGCCAAGCTTCGTTGCTAGTATCAATTCGCTAATTATACACCTGCCAGAGGATACA
            Promoteur et Site de fixation des ribosomes de PAM

ATG AAA AAT AGA AAT CGT ATG ATC GTG AAC TGT GTT ACT GCT TCC CTG
Met Lys Asn Arg Asn Arg Met Ile Val Asn Cys Val Thr Ala Ser Leu
«........séquence signal de la PAM...............................
                      <————séquence délétée pour construire

ATG TAT TAT TGG AGC TTA CCT GCA CTG GCT GAT GCA CAC AAG...
Met-Tyr Tyr Trp Ser Leu Pro Ala Leu Ala Asp Ala His Lys...
..........................................»«.....SAH..........
PAM1-SAH————————————————————————>

Séquence des signaux d'expression et du début de la fusion
"Peptide signal PAM-SAH" de pXL288.

Figure 9

A. SEQUENCES DES ACIDES AMINES DES DIFFERENTS SEGMENTS "PSEUDO-PRO"

cII-SAH:
```
MET VAL ARG ALA ASN LYS ARG-ASP
ATG GTT CGT GCA AAC AAA CGC GAT..
                              aa 1 SAH
```

PAM1:
```
MET LYS ASN ARG ASN ARG-ASP
ATG AAA AAT AGA AAT CGT GAT....
```

PAM2:
```
MET LYS ASN ARG LYS ARG-ASP
ATG AAA AAT AGA AAA CGT GAT....
```

PAM3:
```
MET LYS LYS ARG LYS ARG-ASP
ATG AAA AAA AGA AAA CGT GAT...
```

B. MODIFICATIONS EFFECTUEES SUR PAM1

PAM3                                LYS        LYS}

PAM2                                           LYS}

PAM1                    MET LYS ASN ARG ASN ARG ASP
                       ATG AAA AAT AGA AAT CGT GAT....

                                    A} modifications effectuées
                                    }par mutagénèse dirigée
                                 A}par oligonucléotide

Figure 10

39

A. OLIGONUCLEOTIDE UTILISE POUR LA MUTAGENESE PAR DELETION POUR CONSTRUIRE PAM1 - SAH (pXL641)

5'-ATGAAAAATAGAAATCGTGATGCACACAAGAGTG-3'
  PAM                    SAH

B. OLIGONUCLEOTIDE UTILISE POUR LA MUTAGENESE DIRIGEE POUR CONSTRUIRE PAM2 - SAH (pXL740)

5'CAATGAAAAATAGAAAACGTGATGCACACAAGAGT-3'

nucléotide modifié

C. OLIGONUCLEOTIDE UTILISE POUR LA MUTAGENESE DIRIGEE POUR CONSTRUIRE PAM3 - SAH (pXL741)

5'AGGATACAATGAAAAAAGAAAACGTGATGCACACAAGAGT-3'

nucléotides modifiés

Figure 11